# EUROPEAN PATENT APPLICATION

(11) **EP 2 829 279 A1**
(43) Date of publication of application: **28.01.2015**
(21) Application number: 14176850.7
(22) Date of filing: 25.02.2011
(51) Int. Cl.: A61K 38/18, A61K 38/10, A61P 29/00, A61P 37/06, G01N 33/68, C07K 7/08, C07K 14/47, C12N 15/12

(54) **Modulation of cytokine-induced chronic inflammatory responses**

(30) Priority: 25.02.2010 US 308269 P; 09.07.2010 US 362935 P
(62) Divisional of application: 11746785.2
(71) Applicant: University Of Manitoba, Winnipeg, Manitoba R3T 5V4 (CA)
(72) Inventor: Mookherjee, Neeloffer, Winnipeg, Manitoba R3T 5V4 (CA)
(74) Representative: Nederlandsch Octrooibureau

(57) **Abstract**

A composition for treatment of a chronic inflammation condition or an autoimmune disorder wherein the composition comprises a therapeutically effective amount of a polypeptide molecule comprising an amino acid sequence that shares at least 80% sequence identity with an amino acid sequence selected from SEQ ID NO: 22, and SEQ ID NO 23. A composition for treatment of a chronic inflammation condition or an autoimmune disorder wherein the composition comprises a therapeutically effective amount of an analog of a LL-37 peptide molecule. A composition for treatment of a chronic inflammation condition or an autoimmune disorder wherein the composition comprises a therapeutically effective amount of an exogenous agonist for increasing cellular production and/or cellular activation and/or cellular expression of a LL-37 peptide. Use of one or more of the compositions for treatment of a chronic inflammation condition or an autoimmune disorder.

## Description

### TECHNICAL FIELD

The present invention relates to methods for diagnosing or monitoring a chronic inflammation condition or an autoimmune disorder. The invention also relates to compositions and use of the compositions for treating a chronic inflammation condition or an autoimmune disorder.

### BACKGROUND

Breakdown of the balance between pro-inflammatory and anti-inflammatory responses and the dysregulation of the inflammatory cascade is a major contributing factor in the development of chronic inflammatory diseases including arthritis. In recent years cytokines IL-17 and IL-32 have been suggested to contribute to the chronicity of inflammatory diseases including arthritis. The levels of both IL-17 and IL-32 are elevated in arthritic synovium, and these cytokines are inducers of critical pro-inflammatory cytokines such as TNF and IL-1. An important distinction between these two cytokines is that the role of IL-17 in the sustenance and escalation of arthritic inflammatory conditions has been suggested to be TNF-independent, and in contrast, the role of IL-32 in inducing joint inflammation is through a TNF-dependent mechanism. In arthritic patients on biologic therapeutics targeting TNF, the arthritic condition is not completely controlled and some patients do not respond to anti-TNF therapy.

### SUMMARY

Some embodiments of the present invention relate to methods for diagnosing or monitoring a chronic inflammation condition or an autoimmune disorder wherein the methods include monitoring the response of synovial fibroblasts to interleukin 17 (IL-17) and interleukin 32 (IL-32). Stimulation of IL-32 results in phosphorylation of the TNF receptor. Some aspects of the present invention relate to methods for monitoring levels of TNF receptor and/or P300 and/or DAPK in physiological sample collected from subjects. Some aspects relate to kits for detecting levels of TNF receptor and/or P300 and/or DAPK in a physiological sample collected from a subject. The kits may comprise antibodies specific to at least one of TNF receptor or subcomponents thereof, P300 or subcomponents thereof, and DAPK or subcomponents thereof. The kits may optionally comprise components having therein or thereon antibodies specific to at least one of TNF receptor or subcomponents thereof, P300 or subcomponents thereof, and DAPK or subcomponents thereof.

Some embodiments of the present invention relate to compositions for treatment of a chronic inflammation condition or an autoimmune disorder. It has been found that the LL-37 peptide can modulate the production and/or expression of IL-32 thereby interfering with phosphorylation of the TNF receptor and subsequent production of and/or activation of P300 or DAPK. Some aspects of the present invention relate to exogenous agonists for increasing cellular production and/or expression of the LL-37 peptide in a subject. Suitable exogenous agonists are exemplified by small molecule chemicals selected by a screening process for identification of chemical compounds that interfere with and/or inhibit phosphorylation of the TNF receptor and subsequent production of and/or activation of P300 or DAPK. Some aspects of the present invention relate to analogs of the LL-37 peptide and compositions comprising analogs of the LL-37 peptide. Some aspects of the present invention relate to use of the exogenous agonists and/or the LL-37 peptide analogs for treating a chronic inflammation condition or an autoimmune disorder. Some aspects of the present invention relate to exogenous agonists for inhibiting phosphorylation of the TNF receptor and/or production of and/or activation of P300 or DAPK.

### BRIEF DESCRIPTION OF THE DRAWINGS

The present invention will be described in conjunction with reference to the following drawings, in which:
Fig. 1 is a schematic illustration showing the interactions between cellular populations and pro-inflammatory mediators resulting in inflammation responses;
Fig. 2 is a schematic illustration showing the effects of the LL-37 on the interactions between cellular populations and pro-inflammatory mediators;
Figs. 3A-3C show chemokine production by human monocytic cell line THP-1 *(in vitro)* and human FLS cells isolated from synovial tissues *(ex vivo)* induced in the presence of IL-17 and IL-32;
Figs. 4 show transcriptional responses induced in the presence of IL-17 and IL-32 in human monocytic cell line THP-1 (4A), and human FLS cells (4B);
Figs. 5A and 5B show the effects of IL-32 and IL-17 on phosphorylation of p300 and DAPK1 by human monocytic cells;
Fig. 6A shows differential modulation of IL-17- and IL-32-induced responses in the presence of immune modulator TNF-α, Figs. 6B-6E show differential modulation of IL-17- and IL-32-induced responses in the presence of immune modulators TNF-α and human host defence peptide LL-37;
Figs. 7A-7D show the effects of IL-17, IL-32, IL-1β, TNF-α, and LPS on knockdown of p300 and DAPK-1 in human macrophage-like THP-1 cells (7A) and on mRNA expression (7B). Tissue culture supernatants were monitored for the production of chemokine IL-8 (7C) and cytokine TNF-α (7D);
Figs. 8A and 8B show the effects of IL-17 and IL-32 on transcriptional responses in human FLS cells (8A) or macrophage-like THP-1 cells (8B);
Fig. 9 shows differential stimulation of phosphorylation by IL-17 and IL-32. THP-1 cells stimulated with either IL-17 or IL-32;
Figs. 10A and 10B show the effects of IL-17 and IL-32 on transcriptional responses in human macrophage-like THP-1 cells (10A) and human FLS cells isolated from synovial tissues (10B);
Fig. 11 shows the effects of IL-17 and IL-32 on activation of transcription factor NF-κB in synovial fibroblasts produced by rabbit synovial fibroblast cell line HIG-82;
Figs. 12A-12D show the effects of the TNF-R1 monoclonal antibody on the IL-17 and IL-32-stimulated stimulated production of cytokines in human macrophage-like THP-1 cells. Transcriptional responses were evaluated for chemokine Gro-α (12A)and pro-inflammatory cytokine IL-23 (12B). Tissue culture supernatants were monitored after 24 hr of stimulation for the production of chemokine Gro-α (12C) and chemokine IL-8 (12D);
Figs. 13A-13D show the effects of IL-17, IL-32, IL-1β, TNF-α, and LPS on knockdown of p300 and DAPK-1 in human macrophage-like THP-1 cells (13A) and on mRNA expression (13B). Tissue culture supernatants were monitored for the production of chemokine IL-8 (13C) and cytokine TNF-α (13D);
Figs. 14A-14C show the effects of IL-17 and IL-32 on the mediation of activation transcription factor NF-κB in human FLS cells (14A) and macrophage-like THP-1 cells (14B). were stimulated with cytokines either IL-17, IL-32, TNF-α or IL-1β for 30 min. Fig. 14C shows the effects of IL-17, IL-32 and TNF-α on activation of NF-κB in rabbit synovial fibroblast cell line HIG-82;
Fig. 15 shows the effects of TNF-α on modulation of inflammatory responses induced by IL-17 and IL-32 in human FLS cells;
Figs. 16A and 16B shows the effects of LL-37 on transcriptional responses induced by IL-17 and IL32in macrophage-like THP-1 cells (16A) and human FLS cells (16B);
Figs. 17A and 17B show the effects of LL-37 on protein production induced by IL-32. Tissue culture supernatants were monitored for pro-inflammatory cytokines TNF-α and IL-1β (17A) and chemokines IL-8 and GROα (17B);
Figs. 18A and 18B show the effects of peptides derived from LL-37 on protein production induced by IL-32 by human macrophage-like THP-1 cells. Tissue culture supernatants were monitored for pro-inflammatory cytokines TNF-α and IL-1β (18A) and chemokines IL-8 and GROα (18B);
Figs. 19A and 19B show the effects of TNF-α and IL-1β on production of IL-8 by THP-1 cells. TC supernatants were monitored for production of IL-8 (19A). Cell lysates were treated with a biotin alkyne reagent, thus covalently linking a biotin alkyne tag to the azide reactive group of the AHA-containing nascent proteins (19B);
Figs. 20A-20D show the effects of TNF-α and IL-1β on gene expression; and
Fig. 21 shows suppression of IL-32induced pro-inflammatory protein production by LL-37 and its synthetic derivations IG-25, IG-19, and RK-25 in peripheral blood mononuclear cells.

### DETAILED DESCRIPTION

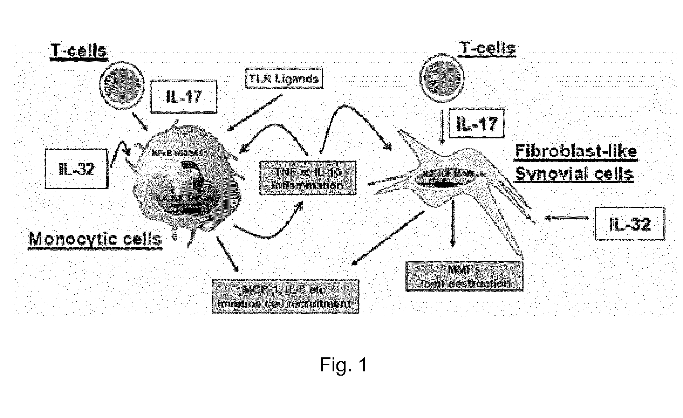

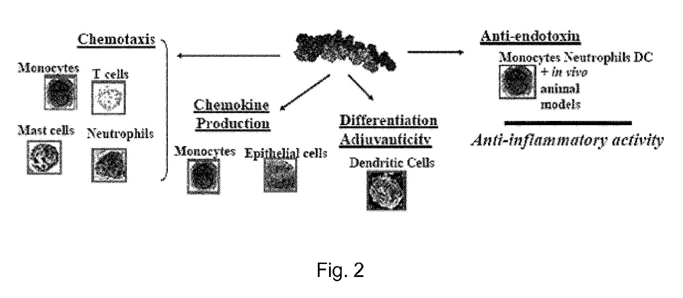

Two recently discovered pro-inflammatory cytokines, IL-32 and IL-17, are associated with the pathogenesis of chronic inflammatory and autoimmune diseases. IL-32 (formerly known as natural killer transcript 4), is a pro-inflammatory cytokine, found in activated T-cells, NK cells, and monocytic cells and is a potent inducer of pro-inflammatory mediators such as TNF-α. IL-32 .is found to be significantly elevated in rheumatoid arthritic synovial tissues and can induce joint inflammation and cartilage damage as well as osteoclast differentiation (Fig. 1). Similarly, IL-17 is a pro-inflammatory cytokine primarily synthesized by T-lymphocytes and is a potent inducer of TNF-α, IL-1β and matrix metalloproteinases, contributing to the inflammatory process and cartilage damage in arthritis (Fig. 2). I hypothesized that the common targets (if any) of IL-32 and IL-17 may represent critical nodes or hubs within the different inflammatory networks that may or may not be TNF-dependent. Identification of such targets could be especially important for non-responders of current pharmacological treatments, and would serve as viable candidates for further investigation of alternate intervention strategies for diseases characterized by chronic inflammation.

Assessments were made of molecular responses (protein production, gene expression and signal transduction) induced by IL-32 and IL-17 in two critical cell types contributing to arthritis; monocytic cells and fibroblast-like synovial (FLS) cells. Overall phosphorylation profiles induced by IL-32 and IL-17 were largely different. In addition, cellular responses induced by IL-32 and IL-17 in the presence of other molecules that are elevated in sites of inflammation, such as cytokine TNF-α or human cathelicidin peptide LL-37, were differentially altered. Taken together, these results indicated that the cytokines IL-32 and IL-17 may be employing different regulatory processes. The kinome screen revealed that the TNF-receptor-1 (TNF-R1) was phosphorylated in the presence of IL-32 but not IL-17. These are the first studies to demonstrate that IL-32-induced downstream response was indeed dependent on TNF-R1. These results provided an insight into the molecular mechanism of the TNF-dependent role of IL-32 in inflammation, which may be due to the engagement of TNF-R1 by IL-32.

In spite of the evidences suggesting differential regulatory processes employed by cytokines IL-32 and IL-17, a kinome screen revealed two common phosphorylation targets of IL-32 and IL-17, namely the transcriptional co-activator p300 and death-associated protein kinase (DAPK). These are the first studies to demonstrate that both p300 and DAPK1 are phosphorylated in the presence of the cytokines IL-32 or IL-17. The presence of common targets is further supported by observations that IL-32 and IL-17 induced some similar downstream responses such as induction of chemokines Gro-α and IL-8 production, or the direct activation of transcription factor NF-κB. Since IL-32 and IL-17-induced inflammatory responses may be differentially dependent on the TNF-pathway, the identified common protein targets represent overlapping hubs within the inflammatory network for both TNF-dependent and-independent processes.

### EXAMPLES

### Example 1:

### Materials and Methods

***Cell culture and isolation:*** Human fibroblast-like synovial (FLS) cells were isolated from synovial tissues obtained from patients with. Briefly, the tissues were digested with 1 mg/ml collagenase and 0.05 mg/ml hyaluronidase (both obtained from Sigma; Oakville, ON, Canada) in Hanks' balanced salt solution (Gibco) for 1-2 hours at 37°C. The cells were cultured in DMEM media (Gibco) supplemented with sodium pyruvate and non-essential amino acids (referred to as complete DMEM media henceforth), containing 10% in a humidified incubator at 37°C and 10% CO₂. A rabbit synoviocyte cell line HIG-82 (ATCC^{®} CRL-1832; ATCC is a registered trademark of the American Type Culture Corp., Manassas, VA, USA) was cultured in Ham's F-12 growth medium containing glutamine (Gibco; Invitrogen Canada Inc., Burlington, ON, Canada) supplemented with sodium pyruvate (referred to as complete F-12 media henceforth), containing 10% (v/v) fetal bovine serum (FBS) in a humidified incubator at 37°C and 5% CO₂. Confluent HIG-82 or human FLS cells were trypsinized with 1:3 dilution of 0.5% trypsin-EDTA (Invitrogen) in Hanks' balanced salt solution. The synoviocytes (either HIG-82 or human FLS cells) were seeded at 2 X 10⁴ cells/ml, either 1 ml per well in 24-well tissue culture plate, 0.5 ml per well in 48-well tissue culture plate, or 3 ml per well in 6-well tissue culture plate as required. The synoviocytes were propagated in their respective complete media containing 10% (v/v) FBS overnight. The following day, the culture media was changed to complete media containing 1% (v/v) FBS before the addition of the various stimulants. Human monocytic THP-1 (ATCC^{®} TIB-202) cells were cultured in RPMI-1640 media containing 2 mM L-glutamine and 1 mM sodium pyruvate (referred to as complete RPMI media from here on), supplemented with 10% (v/v) FBS, and maintained in a humidified incubator at 37 °C and 5% CO₂. The THP-1 cells were differentiated to plastic-adherent macrophage-like cells by treatment with phorbol 12-myristate 13-acetate (PMA; Sigma-Aldrich Canada, Oakville, ON). Cellular cytotoxicity was evaluated after 24 hr stimulation with the various stimulants used in this study by monitoring the release of lactate dehydrogenase (LDH) employing a colorimetric detection kit (Roche Diagnostics, Laval, QC, Canada).

***Stimulants, reagents and antibodies:*** Recombinant human cytokines TNF-α, IL-1β, IL-17A/F (referred to as IL-17 here on) and IL-32 were all obtained from eBioscience, Inc (San Diego, CA, USA). Human cationic host defence peptide LL-37 was synthesized and obtained from CPC Scientific Inc., (San Jose, CA, USA). The peptide was re-suspended in endotoxin-free water and stored at -20°C until further use. Anti-human monoclonal antibodies directed against TNFR; TNFR-1 and TNFR-2 (MAB625 and MAB726 respectively) were obtained from R&D Systems Inc., (Minneapolis, MN, USA). Phospho-p300 (Ser1834) antibody was obtained from MJS Biolynx (Brockville, ON, Canada,). Antibody directed against phospho-DAPK (Ser308) was obtained from Santa Cruz Biotechnology Inc., (Santa Cruz, CA, USA). Monoclonal antibody to glyceraldehydes-3-phosphate dehydrogenase (GAPDH), and HRP-linked purified anti-rabbit IgG and anti-mouse IgG secondary antibodies were all from Cell Signaling Technology, distributed by New England Biolabs Ltd., (Pickering, ON, Canada). ***ELISA immunoassay:*** Tissue culture supernatants were centrifuged at 1500 X g for 5-7 min to obtain cell-free samples and the aliquots were stored at -20°C until further use. Production of chemokine Gro-α was monitored in the tissue culture supernatants by ELISA employing human Gro-α DuoSet (R&D Systems Inc.) as per the manufacturer's instructions. Production of cytokine TNF-α was monitored in the tissue culture supernatants using specific antibody pairs from eBioscience, Inc., and production of IL-8 was monitored using specific antibody pairs from R&D Systems, Inc., as per the manufacturer's instructions. The concentration of the cytokines or chemokines in the tissue culture supernatants was evaluated by establishing a standard curve with serial dilutions of the recombinant human cytokines or chemokines as required.

***Quantitative real-time PCR (qRT-PCR):*** Human FLS or monocytic THP-1 cells were stimulated with indicated stimulants for 4 hr. RNA was isolated and analyzed for gene expression by qRT-PCR using SuperScript III Platinum Two-Step qRT-PCR Kit with SYBR Green (Invitrogen), according to the manufacturer's instructions, in the ABI PRISM 7000 sequence detection system (Applied Biosystems). Fold changes were calculated using the comparative Ct method, after normalization using 18S rRNA primers. The list of primers employed is shown in Table 1.

**Table 1:**

| Phosphoprotein | ID | Kinase Target | IL-17 Relative Fold change | *P*-value | IL-32 Relative Fold change | *P*-value |
|---|---|---|---|---|---|---|
| STMN1 | P16949 | S15 | -2.02 | 0.048346 | 4.94 | 0.119778 |
| TNF-R1 | P19438 | S274 | -1.17 | 0.014856 | 4.70 | 0.006443 |
| VEGFR3 | P35916 | Y1265 | -1.00 | 0.001768 | 3.69 | 0.093431 |
| PLCG1 | P19174 | Y472 | -2.09 | 0.041804 | 3.54 | 0.016014 |
| Fyn | P06241 | Y420 | -1.35 | 0.003994 | 3.43 | 0.014261 |
| CTSB | P07858 | Y219 | 1.34 | 0.23606 | 3.27 | 0.00709 |
| Akt1 | P31749 | | 1.11 | 0.21268 | 3.26 | 0.004067 |
| Rb | P06400 | S780 | -1.92 | 0.001168 | 3.23 | 0.009717 |
| Cdc42 | P60953 | Y64 | 1.25 | 0.313626 | 3.21 | 0.005469 |
| MKP-1 | P28562 | S359 | -1.00 | 0.001034 | 2.51 | 0.010586 |
| SRF | P11831 | S77/79 | -1.87 | 0.198335 | 1.97 | 0.028672 |
| Fyn | P06241 | Y531 | -1.35 | 0.284851 | 1.81 | 0.056901 |
| caveolin-1 | Q2TNI1 | Y14 | -3.51 | 0.072589 | 1.74 | 0.043075 |
| p300 | Q09472 | S1834 | 1.92 | 0.002546 | 1.71 | 0.05587 |
| PPARG | P37231 | S112 | -2.41 | 0.06577 | 1.63 | 0.021592 |
| FRS2 | Q8WU20 | Y196 | -2.36 | 0.076686 | 1.62 | 0.024022 |
| MEK1 | Q02750 | S217 | -2.47 | 0.101358 | 1.50 | 0.013693 |
| VIM | P08670 | S39 | -1.82 | 0.270624 | 1.48 | 0.056439 |
| DAPK1 | P53355 | S308 | 2.19 | 0.007522 | 1.45 | 0.047372 |
| NGFR | P08138 | Y336 | -2.93 | 0.193009 | 1.37 | 0.016714 |
| APE1 | P27695 | S289 | 1.52 | 0.026386 | 1.28 | 0.063852 |
| 4E-BP1 | Q13541 | T46 | 1.77 | 0.001258 | -1.00 | 0.005399 |
| IkB-beta | Q15653 | S313/15 | -1.92 | 3.60E-04 | -1.22 | 0.033048 |
| EphA2 | P29317 | Y772 | -1.88 | 0.029456 | -1.22 | 0.075177 |
| FRS3 | Q8WU20 | Y416 | -4.37 | 0.025056 | -1.26 | 0.144433 |
| MKP-1 | P28562 | S296 | -8.08 | 0.040502 | -1.49 | 0.076952 |
| gp130 | P40189 | Y767 | -5.91 | 0.046097 | -1.60 | 0.002387 |
| ROCK2 | 075116 | S1134/7 | 1.20 | 0.001374 | -1.60 | 0.008068 |
| PKACa | P17612 | S338 | -1.64 | 5.46E-04 | -1.64 | 0.007801 |
| STAT2 | P52630 | Y690 | -1.05 | 0.185679 | -1.67 | 0.021569 |
| CREB | P16220 | S110/1 | -2.92 | 6.49E-04 | -1.68 | 0.010216 |
| Fas | P25445 | Y291 | -1.07 | 0.244763 | -1.74 | 0.022505 |
| Kit | P10721 | Y721 | -1.75 | 0.001468 | -1.75 | 0.008233 |
| Cystatin S | P01036 | S132 | -2.33 | 5.08E-04 | -1.78 | 0.003754 |
| Grb10 | Q13322 | S150 | -1.86 | 0.001074 | -1.86 | 0.009181 |
| Akt1 | P31749 | | -7.78 | 0.018698 | -1.87 | 0.171965 |
| Fos | P01100 | T232 | 1.51 | 0.020475 | -1.92 | 0.007536 |
| STAT6 | P42226 | Y641 | -1.94 | 0.001601 | -1.94 | 0.012476 |
| TAK1 | 043318 | T184 | -2.01 | 0.001519 | -2.01 | 0.006928 |
| NFAT4 | Q12968 | S163/5 | -2.22 | 0.001339 | -2.22 | 0.0083 |
| MEK5 | Q13163 | S311/15 | -6.20 | 0.009408 | -2.29 | 0.001913 |
| Smad3 | P84022 | S422/3/5 | -2.37 | 0.001995 | -2.37 | 0.008028 |
| p53 | P04637 | S6/9 | -2.43 | 0.001053 | -2.43 | 0.00479 |
| NFkB-p100 | Q00653 | S865 | -1.03 | 0.006376 | -2.54 | 0.008275 |
| CCR5 | P51681 | S336/7 | -2.62 | 0.001757 | -2.62 | 0.004969 |
| CaMK2-alpha | Q9UQM7 | T286 | -1.60 | 3.59E-04 | -3.12 | 0.006745 |
| SEK1 | P45985 | S80 | 1.00 | 0.083006 | -3.25 | 0.010964 |
| Akt1 | P31749 | S124/9 | -3.27 | 0.001009 | -3.27 | 0.011844 |
| Daxx | Q9UER7 | S668/71 | -3.45 | 0.00205 | -3.45 | 0.009993 |
| NFkB-p105 | P19838 | S337 | -3.16 | 0.180242 | -3.63 | 0.009452 |
| axin-1 | 015169 | S486 | -3.73 | 7.34E-04 | -3.73 | 0.003599 |
| Src | P12931 | S74 | -6.77 | 0.00113 | -3.78 | 0.005773 |
| CTNNB1 | P35222 | T41/45 | -4.12 | 9.09E-04 | -4.12 | 0.008661 |
| FLGE_ECOLI | Q8X8L1 | | -2.37 | 0.015468 | -4.18 | 0.004231 |
| FGFR1 | P11362 | Y154 | -2.32 | 0.103795 | -9.59 | 0.005387 |
| BLNK | Q8WV28 | Y96 | 1.14 | 0.009763 | -2.29 | 0.010163 |

***Kinome profiling:*** Human monocytic THP-1 cells were differentiated to plastic-adherent macrophage-like cells by treatment with PMA as described above, the cells were rested for 24 hr and then stimulated with either IL-32 (20 ng/mL) or IL-17 (20 ng/mL) for 15 min. Subsequently, cellular lysates were prepared in lysis buffer (containing 20mM Tris-HCl pH 7.5, 150 mM NaCl, 1 mM EDTA, 1mM EGTA, 1 mM sodium fluoride, 1mM sodium orthovanadate, 2.5 mM sodium pyrophosphate, protease inhibitor cocktail (Sigma-Aldrich) and 1% (v/v) Triton X-100, as taught by Jalal et al. (2009, Genome to kinome: species-specific peptide arrays for kinome analysis. Sci. Signal 2:11). Peptide arrays representing phosphorylation targets of kinase activity were incubated with the cellular lysates for quantifying global kinase activity following the methods disclosed by Jalal et al. (2009). The trends of protein phosphorylation events upon stimulation with IL-32 and IL-17 were comprehensively analysed after normalization and background correction of signal strength following the methods disclosed by Jalal et al. (2009). The phosphorylations of the peptides on the array were quantitated in the cytokine-treated cells relative to the un-stimulated control cells. Differentially phosphorylated targets were defined as ≥ 1.45 fold increase or decrease *(p* < 0.06) in phosphorylation compared to un-stimulated control cells.

***Immunoblots:*** Cell lysates were prepared in lysis buffer containing 20mM Tris-HCl pH 7.5, 150 mM NaCl, 1 mM EDTA, 1mM EGTA, 1 mM sodium fluoride, 1mM sodium orthovanadate, 2.5 mM sodium pyrophosphate, protease inhibitor cocktail (Sigma-Aldrich) and 1% (v/v) Triton X-100. The lysates were electrophoretically resolved on a 4% - 12% NUPAGE^{®} Bis-Tris gels (Invitrogen Corporation, Burlington, ON, Canada; NUPAGE is a registered trademark of Life Technologies Corp., Carlsbad, CA, USA), followed by transfer to nitrocellulose membranes (Millipore, Canada). The membranes were subsequently probed with either phospho-p300 (Ser1834) polyclonal antibody, monoclonal antibody against phospho-DAPK (Ser308) or monoclonal antibody to glyceraldehyde-3-phosphate dehydrogenase (GAPDH) in TBST (20 mM Tris pH 7.5, 150 mM NaCl, 0.1% Tween 20) containing 5% skimmed milk powder. Affinity purified HRP-linked horse anti-mouse or goat anti-rabbit antibodies were used for detection as required. The membranes were development with Amersham ECL detection system (GE Healthcare, Baie d'Urfe QC, Canada) according to the manufacturer's instructions.

***NF-κB-reporter luminensence assay:*** Rabbit synoviocyte HIG-82 cells were transiently transfected with pNFκB-MetLuc2-Reporter Vector (Clontech laboratories Inc., Mountain View, CA, USA) or the provided control vector as per the manufacturer's instructions. Various stimulants were added to the transfected cells in culture media containing 1% (v/v) FBS. The cells were stimulated with either recombinant human IL-32 or IL-17, and in parallel with known activators of NF-κB such as pro-inflammatory recombinant human cytokines TNF-α □ and IL-1β, or bacterial lipopolysaccharide (LPS), for 4 or 6 hr. The activation of NF-κB was monitored by employing the Ready-To-Glow Secreted NF-κB Luciferase Reporter Assay (Clontech) as per the manufacturer's instructions.

### Results

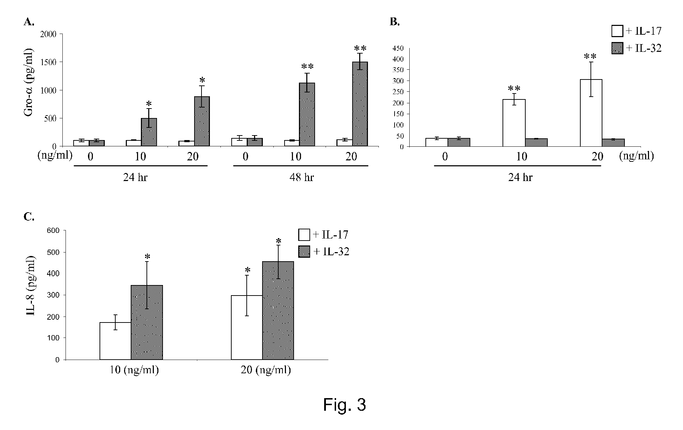

***IL-32- and IL-17-induced chemokine production and transcriptional responses in human monocytic and FLS cells:*** Human monocytic THP-1 cell line *(in-vitro)* and human FLS cells *(ex-vivo)* were stimulated with either recombinant human IL-32 or IL-17 for the indicated time points. The tissue culture supernatants were monitored for the production of chemokine Gro-α or IL-8 by ELISA using specific antibodies (eBiosciences). There was a robust induction of the chemokine Gro-α production by IL-32 in human monocytic cells (Fig. 3A), and by IL-17 in FLS cells (Fig. 3B). However, both the cytokines, IL-32 and IL-17, induced the similar production of chemokine IL-8 in human THP-1 monocytic cells after 24 hr of stimulation (Fig. 3C).

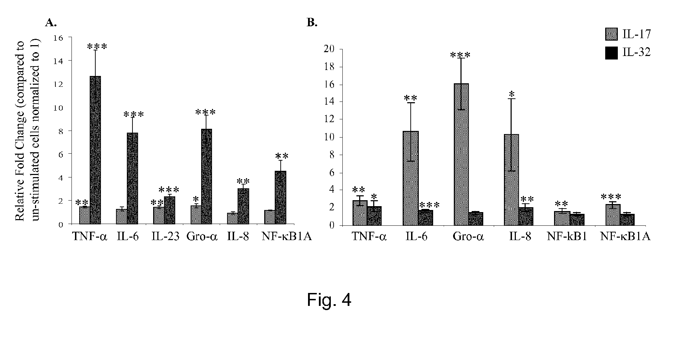

Transcriptional responses were investigated after stimulation of monocytic THP-1 cells and human FLS cells with either IL-32 or IL-17 (20 ng/mL each) for 4 hr. RNA was isolated and analyzed for gene expression of inflammatory cytokines TNF-α, IL-6, IL-23, genes encoding for chemokines Gro-α (CXCL1) and IL-8 (CXCL8), and gene expression of NF-κB1 and NF-κB1A (encoding for NF-κB inhibitory protein) by quantitative real-time PCR (qRT-PCR). Even though both IL-32 and IL-17 induced significant (p < 0.05) transcriptional responses in both monocytic and FLS cells, gene expression was largely quantitatively different; cytokine IL-32-induced a quantitatively greater gene expression of pro-inflammatory TNF-α, IL-6 and chemokines Gro-α and IL-8 genes when compared to that induced by IL-17 in human monocytic cells (Fig. 4A). Whereas IL-17-induced gene expression of IL-6, Gro-α and IL-8 were quantitatively greater when compared to that induced by IL-32 in human FLS cells (Fig. 4B). However, expression of IL-32- and IL-17-induced pro-inflammatory IL-23 gene was quantitatively comparable in monocytic cells (Fig. 4A). Similarly, IL-32- and IL-17-induced TNF-α gene expression was comparable in FLS cells (Fig. 4B). Taken together, these results indicated that downstream responses induced in the presence of the cytokines IL-32 and IL-17 in human monocytic and FLS cells appeared to be largely different. However there were some similarities in responses induced by these cytokines in both monocytic and FLS cells.

***Protein phosphorylations induced in the presence of IL-32 and IL-17:*** Protein phosphorylation is a critical mechanism in the regulation of cellular processes. This process is meticulously regulated by enzymes known as kinases, which are increasingly being investigated as drug targets for a variety of diseases. In order to evaluate the regulation of cellular responses in the presence of cytokines IL-32 and IL-17, the global protein phosphorylation events (kinome) employing peptide arrays of specific kinase substrates was investigated. Since both the cytokines IL-32 and IL-17 significantly (p < 0.05) induced transcriptional responses (Fig. 4A), and protein production of chemokine IL-8 in human monocytic THP-1 cell line (Fig. 3C), these cells were employed for a comparative evaluation of the kinome profile in the presence of these cytokines. Human monocytic THP-1 cells were stimulated with either IL-32 (20 ng/mL) or IL-17 (20 ng/mL) for 15 min. Peptide arrays representing phosphorylation targets of kinase activity were employed to comprehensively analyze protein phosphorylation profiles in the presence of these cytokines. The phosphorylations of the peptides on the array were quantitated in the cytokine-treated cells relative to the un-stimulated control cells. The results demonstrated that the overall pattern / trend of phosphorylation events in the presence of the cytokines IL-32 and IL-17 were largely different (Table 2), indicating overall differential kinase activities induced in the presence of these two cytokines. From this analysis, it was noted that cytokine IL-32 significantly induced the phosphorylation of TNF-receptor (TNF-R)-1 by more than four-fold, while the state of TNF-R1 phosphorylation was not altered in the presence of IL-17 as compared to un-stimulated control cells (Table 2). Previous studies have hypothesized that IL-32-mediated pathogenesis in chronic inflammatory diseases may be in part dependent on TNF-α. This is the first study to demonstrate that IL-32-induced direct phosphorylation of TNF-R1.

**Table 2:**

| **Phosphoprotein** | **ID** | **Kinase Target** | **IL-32 Relative Fold change** | ***p*-value** | **IL-17 Relative Fold change** | ***p*-value** |
|---|---|---|---|---|---|---|
| TNF-R1 | P19438 | S274 | 4.70 | 0.006 | -1.17 | 0.014 |
| p300 | Q09472 | S1834 | 1.71 | 0.055 | 1.92 | 0.002 |
| DAPK | P53355 | S308 | 1.45 | 0.047 | 2.19 | 0.007 |

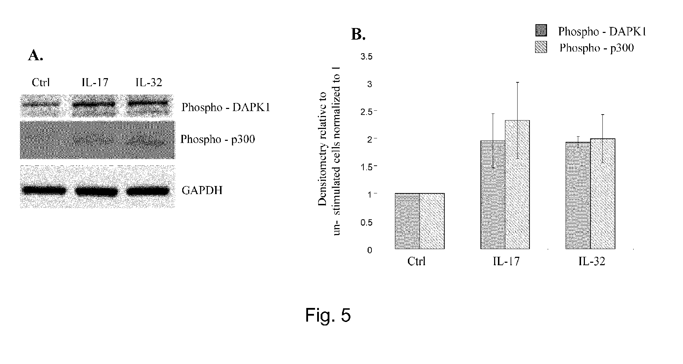

Kinome analysis also revealed that there were two proteins that were significantly phosphorylated in the presence of either IL-32 or IL-17 (Table 2). The transcriptional co-activator p300 and death-associated protein kinase (DAPK) were both phosphorylated by the two cytokines (Table 2). Further probing of immunoblots with specific antibodies to human phospho-p300 (Ser1834) and phospho-DAPK (Ser308) it was conclusively demonstrated that stimulation with either IL-32 or IL-17 resulted in the increased phosphorylation of both p300 and DAPK within 15 min when compared to un-stimulated cells (Fig. 5).

***Differential modulation of cellular responses on stimulation with IL-32 and IL-17 in the presence of other immune mediators:*** Inflammatory responses do not function as a linear cascade of events. It is a complex network of cross-talk between different cellular inflammatory mediators and signalling cascades. The kinome analysis revealed that the phosphorylation profiles induced by IL-32 and IL-17 were largely different. Therefore in order to establish that differential regulatory processes were employed by these two cytokines, this study investigated the impact of other molecules that are found to be elevated at sites of inflammation on IL-32 and IL-17-induced downstream responses.

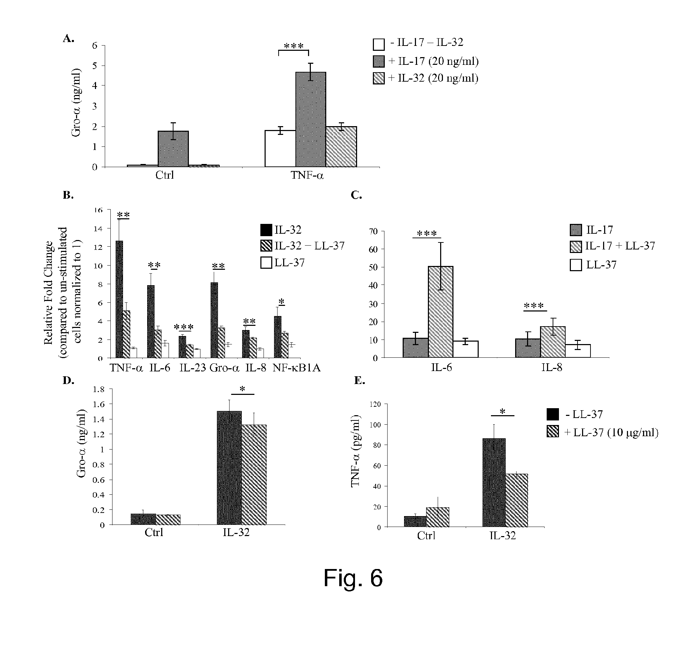

The impact of IL-32 and IL-17-induced responses was assessed in the presence of the two acute inflammatory mediators, TNF-α and IL-1β. Monocytic THP-1 cells and human FLS cells were stimulated with either TNF-α (10 ng/mL) or IL-1β (10 ng/mL) in the presence and absence of either IL-32 or IL-17 (20 ng/mL). The tissue culture supernatants were monitored for the production of chemokines Gro-α and IL-8 by ELISA using specific antibodies. There was no significant alteration of IL-1β-induced chemokine production in either monocytic or FLS cells in the presence of either IL-32 or IL-17 (data not shown). Similarly, no synergistic enhancement of TNF-α-induced chemokine production was observed in the presence either IL-32 or IL-17 in THP-1 monocytic cells (data not shown). In contrast, TNF-α-induced chemokine Gro-α production was significantly (*p* < 0.001) and synergistically enhanced in the presence of IL-17 in human FLS cells after 24 hr of stimulation, but this synergistic effect was not observed in presence of the cytokine IL-32 (Fig. 6A). These results indicated that TNF-α-induced chemokine response was differentially modulated in the presence of cytokines IL-32 and IL-17 in human FLS cells.

The effects of the human cathelicidin LL-37 were assessed on IL-32- and IL-17-induced responses. Cytokine IL-17-induced expressions of pro-inflammatory genes or chemokine genes were not altered in the presence of the peptide LL-37 in human monocytic cells (data not shown). Whereas IL-32-induced gene expression for TNF-α, IL-6 and Gro-α (CXCL1) were all significantly (p < 0.01) suppressed by more that 50%, and the expressions of IL-23, IL-8 (CXCL8) and NF-κB1A were suppressed between 30% - 40%, in the presence of LL-37 in human monocytic THP-1 cells (Fig. 6B). In contrast, some of the IL-17-induced transcriptional responses were significantly enhanced by the presence of the peptide LL-37 in human FLS cells (Fig. 6C). Similar trends were also observed at the protein level on monitoring the tissue culture supernatants for chemokine Gro-α and inflammatory cytokine TNF-α production by ELISA after 24 or 48 hr of stimulation. IL-32-induced chemokine Gro-a production was significantly (p < 0.05) but modestly suppressed by 12 ± 4% (Fig. 6D), and IL-32-induced TNF-α production was suppressed by > 30% (Fig. 6E) in the presence of the peptide LL-37 in human THP-1 monocytic cells. In contrast, IL-17-induced chemokine production was not significantly altered (or modestly enhanced) in the presence of LL-37 in both monocytic THP-1 and human FLS cells (data not shown). These results taken together indicated that IL-32-induced responses were significantly suppressed, and in contrast IL-17-induced immune responses were not suppressed and sometimes enhanced, in the presence of the human cathelicidin peptide LL-37.

The differential trends of alteration of cellular responses on stimulation with IL-32 or IL-17, in the presence of other molecules known to be elevated during inflammation such as cytokine TNF-α or human cathelicidin peptide LL-37 supported the kinome analysis, taken together indicating that the cytokines IL-32 and IL-17 may be engaging different regulatory cellular processes.

These studies were a comparative investigation of IL-32- and IL-17-induced responses in order to provide an insight into the molecular processes, and identified common protein targets for these cytokines. Employing a kinome screen, it was demonstrated that IL-32- and IL-17-induced phosphorylation profiles were overall different, with two common targets. A peptide target corresponding to TNF-receptor-1 (TNF-R1) was phosphorylated by IL-32 but not IL-17. This is the first study to demonstrate that IL-32-mediated downstream responses were dependent on TNF-R1, thus providing an insight into the mechanism for TNF-pathway-dependent role of IL-32 in inflammation. This is the first study to demonstrate the transcriptional co-activator p300 and death-associated protein kinase (DAPK), as common phosphorylation targets of IL-32 and IL-17. Further investigation revealed that both IL-32 and IL-17 induced the activation of NF-κB and chemokine production, indicating similarities in their regulatory processes. The identified common protein targets for cytokines IL-32 and IL-17 represent overlapping nodes within the inflammatory networks of TNF-dependent and-independent processes, and thus are viable as potential therapeutic targets in chronic inflammatory diseases.

### Example 2: Assessment of the effects of IL-17 and IL32 on molecular responses in monocytic cells and fibroblast-like synovial cells

### Materials and Methods

***Cell culture and isolation:*** Human fibroblast-like synovial (FLS) cells were isolated from synovial tissues obtained from patients with osteoarthritis (in accordance to a protocol by the Institutional Review Board at the University of Manitoba) as follows. Briefly, the tissues were digested with 1 mg/ml collagenase and 0.05 mg/ml hyaluronidase (both obtained from Sigma-Aldrich Canada Inc.) in Hanks' balanced salt solution (Gibco; Invitrogen Canada Inc., Burlington, ON, Canada) for 1-2 hours at 37°C. The cells were cultured in DMEM media (Gibco; Canada Inc., Burlington, ON, Canada) supplemented with sodium pyruvate and non-essential amino acids (referred to as complete DMEM media henceforth), containing 10% in a humidified incubator at 37° C and 1% CO₂. A rabbit synoviocyte cell line HIG-82 obtained from the American Type Culture Collection (#CRL-1832; Manassas, VA, USA) was cultured in Ham's F-12 growth medium containing glutamine (Gibco; Invitrogen Canada Inc.) supplemented with sodium pyruvate (referred to as complete F-12 media henceforth), containing 10% (v/v) fetal bovine serum (FBS) in a humidified incubator at 37°C and 5% CO₂. Confluent HIG-82 or human FLS cells were trypsinized with 1:3 dilution of 0.5% trypsin-EDTA (Invitrogen Canada Inc.) in Hanks' balanced salt solution. The synoviocytes (either HIG-82 or human FLS cells) were seeded at 2 X 10⁴ cells/ml, either 1 ml per well in 24-well tissue culture plate, or 0.5 ml per well in 48-well tissue culture plate, or 3 ml per well in 6-well tissue culture plate as required. The synoviocytes were propagated in their respective complete media containing 10% (v/v) FBS overnight. The following day, the culture media were changed to complete media containing 1% (v/v) FBS before the addition of the various stimulants. Human monocytic THP-1 (ATCC^{®} TIB-202; ATCC is a registered trademark of the American Type Culture Collection, Manassas, VA, USA) cells were cultured in RPMI-1640 media containing 2 mM L-glutamine and 1 mM sodium pyruvate (referred to as complete RPMI media from here on), supplemented with 10% (v/v) FBS, and maintained in a humidified incubator at 37°C and 5% CO₂. The THP-1 cells were differentiated to plastic-adherent macrophage-like cells by treatment with phorbol 12-myristate 13-acetate (PMA; Sigma-Aldrich Aldrich Canada Inc.), as taught by Mookherjee et al. (2006, Modulation of the TLR-mediated inflammatory response by the endogenous human host defense peptide LL-37. J. Immunol. 176, 2455-2464). Cellular cytotoxicity was evaluated after 24 hr stimulation with the various stimulants used in this study by monitoring the release of lactate dehydrogenase (LDH) employing a colorimetric detection kit (Roche Diagnostics, Laval, QC, Canada).

***Stimulants, reagents and antibodies:*** Recombinant human cytokines TNF-α, IL-1β, IL-17A/F (referred to as IL-17 here on) and IL-32y (referred to as IL-32 from here on) were all obtained from eBioscience, Inc (San Diego, CA, USA). Human cationic host defence peptide LL-37 was synthesized and obtained from CPC Scientific Inc., (San Jose, CA, USA). The peptide was re-suspended in endotoxin-free water and stored at -20°C until further use. Anti-human monoclonal antibodies directed against TNFR; TNFR-1 and TNFR-2 (MAB625 and MAB726 respectively) were obtained from R&D Systems Inc., (Minneapolis, MN, USA). Phospho-p300 (Ser1834) antibody was obtained from MJS Biolynx (Brockville, ON, Canada,). Antibody directed against phospho-DAPK (Ser308) was obtained from Santa Cruz Biotechnology Inc., (Santa Cruz, CA, USA). Monoclonal antibodies to glyceraldehydes-3-phosphate dehydrogenase (GAPDH), and HRP-linked purified anti-rabbit IgG and anti-mouse IgG secondary antibodies were all obtained from Cell Signaling Technology, distributed by New England Biolabs Ltd., (Pickering, ON, Canada).

***ELISA immunoassay:*** Tissue culture supernatants were centrifuged at 1500 X g for 5-7 min to obtain cell-free samples and the aliquots were stored at -20°C until further use. Production of chemokine Gro-α was monitored in the tissue culture supernatants by ELISA employing human Gro-α DuoSet (R&D Systems Inc.) per the manufacturer's instructions. Production of cytokine TNF-α was monitored in the tissue culture supernatants using specific antibody pairs from eBioscience, Inc., and production of IL-8 was monitored using specific antibody pairs from R&D Systems, Inc., per the manufacturers' instructions. The concentrations of the cytokines or chemokines in the tissue culture supernatants were evaluated by establishing a standard curve with serial dilutions of the recombinant human cytokines or chemokines as required.

***Quantitative real-time PCR (qRT-PCR):*** Human FLS or macrophage-like THP-1 cells were stimulated with indicated stimulants for 4 hr. RNA was isolated and analyzed for gene expression by qRT-PCR using SuperScript III Platinum Two-Step qRT-PCR Kit with SYBR Green (Invitrogen), according to the manufacturer's instructions, in the ABI PRISM 7000 sequence detection system (Applied Biosystems). Fold changes were calculated using the comparative Ct method, after normalization using 18S rRNA primers. The list of primers are shown in Table 3.

**Table 3: Summary of primers used for quantitative real-time PCR.**

| Gene | SEQ ID NO: | Forward primer | SEQ ID NO: | Reverse Primer |
|---|---|---|---|---|
| TNF-α | SEQ ID NO:1 | cagcctcttctccttcctgat | SEQ ID NO:2 | gccagagggctgattagaga |
| CXCL-1 (Gro-α) | SEQ ID NO:3 | tcctgcatcccccatagtta | SEQ ID NO:4 | cttcaggaacagccaccagt |
| IL-6 | SEQ ID NO:5 | caggagcccagctatgaact | SEQ ID NO:6 | gaaggcagcaggcaacac |
| IL-8 | SEQ ID NO:7 | agacagcagagcacacaagc | SEQ ID NO:8 | aggaaggctgccaagagag |
| IL-23 | SEQ ID NO:9 | agcttcatgcctccctactg | SEQ ID NO:10 | ctgctgagtctcccagtggt |
| p300 | SEQ ID NO:11 | gatctgtgtccttcaccatgag | SEQ ID NO:12 | aaacagccatcacagacgaa |
| DAPK-1 | SEQ ID NO:13 | cccagttgaagaacccatagc | SEQ ID NO:14 | cgaggaacattcatgatgtcag |
| 18S RNA | SEQ ID NO:15 | gtaacccgttgaaccccatt | SEQ ID NO:16 | ccatccaatcggtagtagcg |
| NF-κB1 | SEQ ID NO:17 | ctggcagctcttctcaaagc | SEQ ID NO:18 | tccaggtcatagagaggctca |
| NF-κB1A | SEQ ID NO:19 | cagaacaacctgcagcaga | SEQ ID NO:20 | tcagcaatttctggctggtt |

***Kinome profiling:*** Human macrophage-like THP-1 cells were differentiated to plastic-adherent macrophage-like cells by treatment with PMA as described above, after which the cells were rested for 24 hr and then stimulated with either IL-32 (20 ng/mL) or IL-17 (20 ng/mL) for 15 min. Subsequently, cellular lysates were prepared in lysis buffer (containing 20mM Tris-HCl pH 7.5, 150 mM NaCl, 1 mM EDTA, 1mM EGTA, 1 mM sodium fluoride, 1mM sodium orthovanadate, 2.5 mM sodium pyrophosphate, protease inhibitor cocktail (Sigma-Aldrich Canada Inc.) and 1% (v/v) Triton X-100, as taught by Mookherjee et al. (2006). Peptide arrays representing phosphorylation targets of kinase activity were incubated with the cellular lysates for quantifying global kinase activity as taught by Jalal et al. (2009, Genome to kinome: species-specific peptide arrays for kinome analysis. Sci. Signal 2:11). The trends of protein phosphorylation events upon stimulation with IL-32 and IL-17 were comprehensively analysed after normalization and background correction of signal strength as taught by Jalal et al. (2009). The phosphorylations of the peptides on the array were quantitated in the cytokine-treated cells relative to the un-stimulated control cells. Differentially phosphorylated targets were defined as ≥ 1.45 fold increase or decrease (p < 0.06) in phosphorylation compared to un-stimulated control cells.

***Immunoblots:*** Cell lysates were prepared in lysis buffer containing 20mM Tris-HCl pH 7.5, 150 mM NaCl, 1 mM EDTA, 1mM EGTA, 1 mM sodium fluoride, 1mM sodium orthovanadate, 2.5 mM sodium pyrophosphate, protease inhibitor cocktail (Sigma-Aldrich Canada Inc.) and 1% (v/v) Triton X-100. The lysates were electrophoretically resolved on a 4% - 12% NuPAGE^{®} Bis-Tris gels (Invitrogen Canada Inc.) followed by transfer to nitrocellulose membranes (Millipore). The membranes were subsequently probed with either phospho-p300 (Ser1834) polyclonal antibody, monoclonal antibody against phospho-DAPK (Ser308) or monoclonal antibody to glyceraldehyde-3-phosphate dehydrogenase (GAPDH) in TBST (20 mM Tris pH 7.5, 150 mM NaCl, 0.1% Tween 20) containing 5% skimmed milk powder. Affinity purified HRP-linked horse anti-mouse or goat anti-rabbit antibodies were used for detection as required. The membranes were development with Amersham ECL detection system (GE Healthcare, Baie d'Urfe QC, Canada) according to the manufacturer's instructions.

***TNF-receptor blocking assay:*** Human macrophage-like THP-1 cells were differentiated to plastic-adherent macrophage-like cells by treatment with PMA for 24 hr (Sigma-Aldrich Canada Inc.) as taught by Jalal et al. (2009). The cells were rested for an additional 24 hr in complete RPMI media containing 10% (v/v) FBS. Subsequently the media was changed to complete RPMI containing 1% (v/v) FBS. Neutralization or blocking of the TNF-receptors TNFR-1 or TNFR-2 was performed using specific monoclonal antibodies, MAB625 and MAB 726 respectively (R&D Systems Inc) as previously described. Briefly, the cells were pre-incubated with the monoclonal antibodies (20 µg/ml) for 1 hr, followed by treatment with the various stimulants as indicated. RNA was isolated from these cells after 4 hr of stimulation for the evaluation of transcriptional responses employing qRT-PCR, and the tissue culture supernatants were monitored for chemokine Gro-α and IL-8 production after 24 hr of stimulation.

***NF-κB-reporter luminensence assay:*** In order to monitor direct NF-κB activation in synoviocytes, a rabbit synoviocyte cell line, HIG-82 (ATCC^{®} CRL-1832) was transiently transfected with pNFκB-MetLuc2-Reporter Vector (Clontech laboratories Inc., Mountain View, CA, USA) or the provided control vector as per the manufacturer's instructions. Various stimulants were added to the transfected cells in culture media containing 1% (v/v) FBS. The cells were stimulated with either recombinant human IL-32 or IL-17, and in parallel with known activators of NF-κB such as pro-inflammatory recombinant human cytokines TNF-α and IL-1β, or bacterial lipopolysaccharide (LPS), for 4 or 6 hr. The activation of NF-κB was monitored by employing the Ready-To-Glow Secreted NF-κB Luciferase Reporter Assay (Clontech Laboratories Inc.) per the manufacturer's instructions.

***Translocation of NF-κB subunits p50 and p65:*** Nuclear extracts were prepared employing NE-PER extraction reagents (Thermo Fisher Scientific, Toronto, ON, Canada) as per the manufacturer's instructions. In order to monitor direct NF-κB activation, equivalent nuclear extracts (5-8 µg) were resolved on 4% - 12% NUPAGE^{®} Bis-Tris gels and transferred to nitrocellulose membranes. The membranes were blocked with TBST containing 3% fish skin gelatin (Sigma-Aldrich), and probed with antibodies specific for either NF-κB subunit p50 or p65, in TBST containing 1% fish gelatin. The membranes were also probed with antibody to human histone protein HADC-1 to assess the equivalent protein loading. Affinity purified HRP-linked secondary antibodies were used for detection and the membranes were developed with the ECL detection system.

***Gene silencing employing specific siRNA:*** Human macrophage-like THP-1 cells were treated with either Accell SMARTpool siRNA for human EP300, human DAPK1 or non-silencing control in Accell delivery media as per the manufacturer's instructions. After 72 hr the cells were differentiated by PMA treatment. PMA-differentiated plastic-adherent THP-1 cells in the presence and absence of the different siRNA were stimulated with either IL-32 or IL-17 (20 ng/mL each) for the indicated times. RNA was isolated from these cells after 4 hr of stimulation for the evaluation of transcriptional responses, and the tissue culture supernatants were monitored for chemokine Gro-α production after 24 hr of cytokine stimulation.

### Results

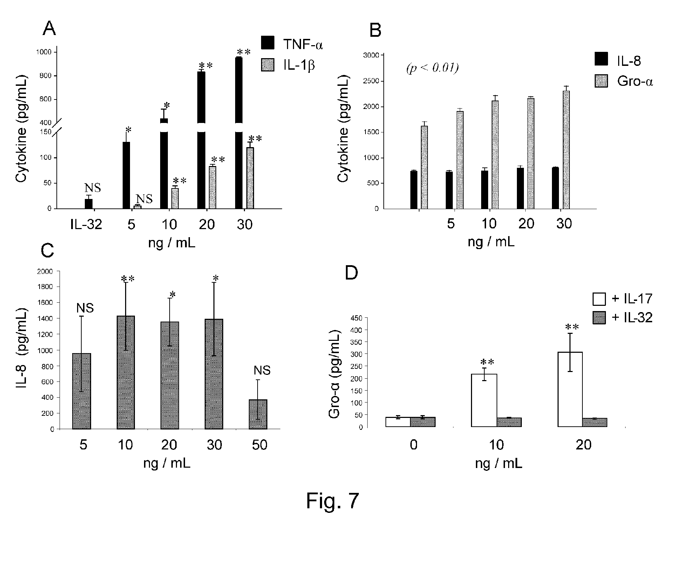

***IL-32- and IL-1 7-induced chemokine production and transcriptional responses in human macrophages and FLS cells:*** In this study, plastic adherent macrophage-like THP-1 cells (*in-vitro*) were stimulated by IL-17 or IL-32 (5 to 100 ng/mL) and the tissue culture (TC) supernatants were monitored for cytokines TNF-α and IL-1β, and chemokines Gro-α and IL-8 by ELISA. There was a dose-dependent production of TNF-α and IL-1β upon stimulation with IL-32, which was significant (p < 0.05) upon stimulation with IL-32 from 20 ng/mL onwards (Fig. 7A). IL-32 also induced significant (p < 0.01) production of chemokines Gro-α and IL-8 from 5 to 100 ng/mL (Fig. 7B), and the amount of chemokine protein produced was similar upon stimulation with 20, 50 or 100 ng/mL. Similarly, there was significant production of IL-8 upon stimulation with 10, 20 or 50 ng/mL of IL-17 *(p* < 0.05), and the amount of IL-8 produced decreased at 100 ng/mL (Fig. 7C). Taken together, these results indicate that both cytokine IL-32 and IL-17 induced downstream protein production in macrophage-like THP-1 cells, and that there was significant (p < 0.05) protein production upon stimulation with either 20 or 50 ng/mL of the cytokines for all the read-outs monitored in this study. We further evaluated protein production in synovial fibroblasts, human FLS cells were stimulated with either IL-32 or IL-17 (10 or 20 ng/mL), and the TC supernatants were monitored for TNF-α, IL-1β and Gro-α production. There was a robust Gro-α production upon IL-17 stimulation in human FLS cells, whereas IL-32 did not produce significant chemokine production (Fig. 7D). In this study, human FLS cells did not produce significant amount of TNF-α or IL-1β upon stimulation with either IL-17 or IL-32 (data not shown). Based on these results, macrophage-like THP-1 cells were selected for a comparative analysis of induced cellular responses upon stimulation with IL-32 and IL-17 at 20 ng/mL.

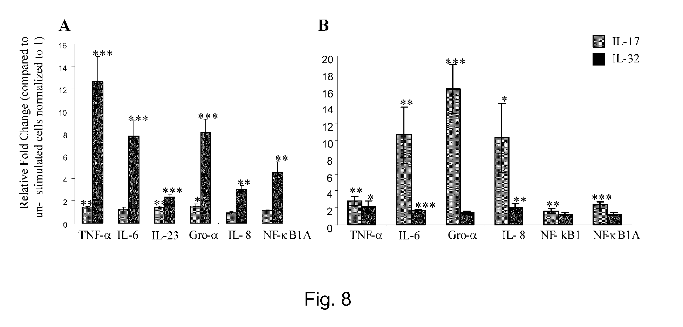

Transcriptional responses were investigated after stimulation of macrophage-like THP-1 cells and human FLS cells with either IL-32 or IL-17 (20 ng/mL each) for 4 hr. RNA was isolated and analyzed for gene expression of inflammatory cytokines TNF-α, IL-6, IL-23, genes encoding for chemokines Gro-α (CXCL1) and IL-8 (CXCL8), and gene expression of NF-κB1 and NF-κB1A (encoding for NF-κB inhibitory protein) by quantitative real-time PCR (qRT-PCR). Even though both IL-32 and IL-17 induced significant (p < 0.05) transcriptional responses in both macrophages and FLS cells, gene expression was largely quantitatively different; cytokine IL-32-induced a quantitatively greater gene expression of pro-inflammatory TNF-α, IL-6 and chemokines Gro-α and IL-8 genes when compared to that induced by IL-17 in human macrophage-like THP-1 cells (Fig. 8A). Whereas IL-17-induced gene expression of IL-6, Gro-α and IL-8 were quantitatively greater when compared to that induced by IL-32 in human FLS cells (Fig. 8B). However, expression of IL-32- and IL-17-induced pro-inflammatory IL-23 gene was quantitatively comparable in macrophage-like THP-1 cells (Fig. 8C). Similarly, IL-32-induced and IL-17-induced TNF-α gene expression was comparable in FLS cells (Fig. 8D). Taken together, these results indicate that downstream responses induced in the presence of the cytokines IL-32 and IL-17 in human macrophages and FLS cells appeared to be largely different. However there were some similarities in responses induced by these cytokines in both macrophages and FLS cells.

***Protein phosphorylations induced in the presence of IL-32 and IL-17:*** Protein phosphorylation is a critical mechanism in the regulation of cellular processes. This process is meticulously regulated by enzymes known as kinases. In order to evaluate the regulation of cellular responses in the presence of cytokines IL-32 and IL-17, we investigated the global protein phosphorylation events (kinome) employing peptide arrays of specific kinase substrates following the methods disclosed by Jalal et al. (2009). Since both the cytokines IL-32 and IL-17 significantly (p < 0.05) induced transcriptional responses (Fig. 8A), and protein production of chemokine IL-8 in human macrophage-like THP-1 cell line (Fig. 7C), these cells were employed for a comparative evaluation of the kinome profile in the presence of these cytokines. Human macrophage-like THP-1 cells were stimulated with either IL-32 (20 ng/mL) or IL-17 (20 ng/mL) for 15 min. Peptide arrays representing phosphorylation targets of kinase activity were employed to comprehensively analyze protein phosphorylation profiles in the presence of these cytokines as taught by Jalal et al. (2009).

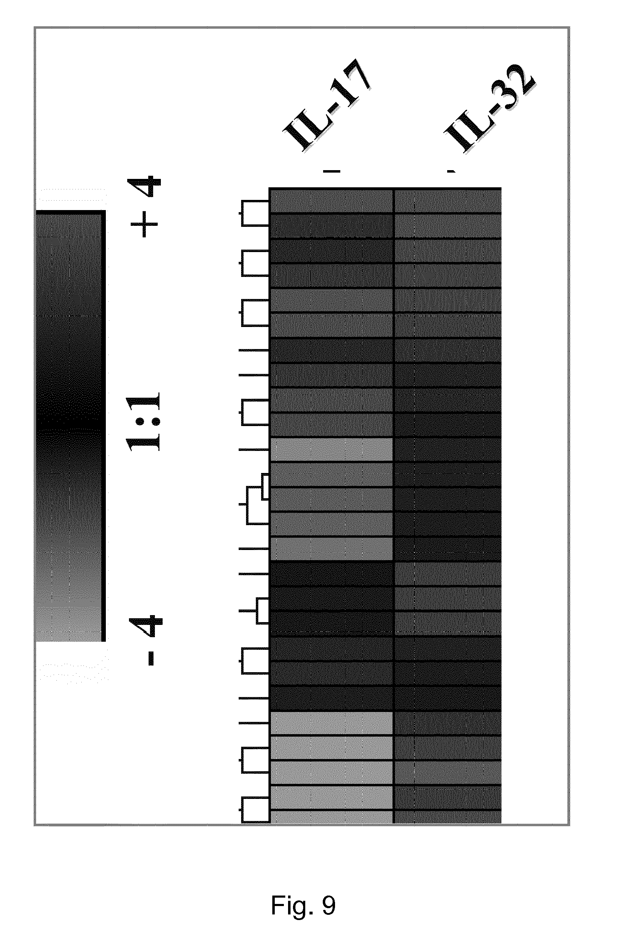

The phosphorylations of the peptides on the array were quantitated in the cytokine-treated cells relative to the un-stimulated control cells. The results demonstrated that the overall pattern / trend of phosphoylation events in the presence of the cytokines IL-32 and IL-17 were largely different (see Fig. 9), indicating overall differential kinase activities induced in the presence of these two cytokines. From this analysis it was noted that cytokine IL-32 significantly induced the phosphorylation of TNF-receptor (TNF-R)-1 by more than four fold, while the state of TNF-R1 phosphorylation was not altered in the presence of IL-17 as compared to un-stimulated control cells (Table 4). This is the first study to demonstrate that IL-32-induced direct phosphorylation of TNF-R1.

**Table 4:**

| **Phosphoprotein** | **ID** | **Kinase Target** | **IL-32 Relative Fold change** | ***p*-value** | **IL-17 Relative Fold change** | ***p*-value** |
|---|---|---|---|---|---|---|
| TNF-R1 | P19438 | S274 | 4.70 | 0.006 | -1.17 | 0.014 |
| p300 | Q09472 | S1834 | 1.71 | 0.055 | 1.92 | 0.002 |
| DAPK | P53355 | S308 | 1.45 | 0.047 | 2.19 | 0.007 |

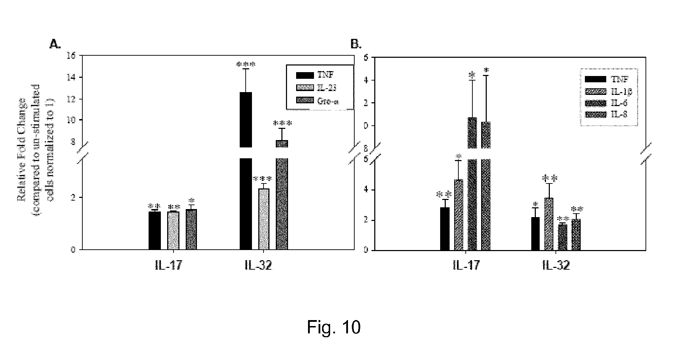

Kinome analysis also revealed that there were two proteins that were significantly phosphorylated in the presence of either IL-32 or IL-17 (Table 4). The transcriptional co-activator p300 and death-associated protein kinase (DAPK) were both phosphorylated by the two cytokines (Table 4). Further probing of immunoblots with specific antibodies to human phospho-p300 (Ser1834) and phospho-DAPK (Ser308) it was conclusively demonstrated that stimulation with either IL-32 or IL-17 resulted in the increased phosphorylation of both p300 and DAPK within 15 min when compared to un-stimulated cells (Fig. 10).

***Differential phosphorylations events in THP-1 cells stimulated by IL-32 and IL-17:*** THP-1 cells were stimulated with either IL-17 (20 ng/ml) or IL-32 (20 ng/ml) for 15 min. The cellular lysates were incubated with peptide arrays representing phosphorylation targets of kinase activity for quantifying global kinase activity as previously described. The phosphorylation of the peptides on the array were quantitated in the cytokine-treated cells relative to the un-stimulated control cells after normalization and background correction of signal strength taught by Jalal et al. (2009). Differentially phosphorylated targets were defined as ≥ 1.5 fold increase or decrease (*p* < 0.06) in phosphorylation compared to un-stimulated control cells (Fig. 9). In the presence of the cytokine IL-17, five peptides on the array (DAPK1, p300, 4E-BP1, APE1 and Fos) demonstrated a > 1.5-fold increase in phosphorylation (*p* ≤ 0.05) relative to un-stimulated cells, which represented the phosphorylation targets for the cytokine IL-17 (Table 5).

**Table 5:**

| **Phosphoprotein** | **ID** | **Kinase Target** | **IL-32 Relative Fold change** | ***P*-value** | **IL-17 Relative Fold change** | ***P***-**value** |
|---|---|---|---|---|---|---|
| STMN1 | P16949 | S15 | -2.02 | 0.048346 | 4.94 | 0.119778 |
| TNF-R1 | P19438 | S274 | -1.17 | 0.014856 | 4.70 | 0.006443 |
| VEGFR3 | P35916 | Y1265 | -1.00 | 0.001768 | 3.69 | 0.093431 |
| PLCG1 | P19174 | Y472 | -2.09 | 0.041804 | 3.54 | 0.016014 |
| Fyn | P06241 | Y420 | -1.35 | 0.003994 | 3.43 | 0.014261 |
| CTSB | P07858 | Y219 | 1.34 | 0.23606 | 3.27 | 0.00709 |
| Akt1 | P31749 | | 1.11 | 0.21268 | 3.26 | 0.004067 |
| Rb | P06400 | S780 | -1.92 | 0.001168 | 3.23 | 0.009717 |
| Cdc42 | P60953 | Y64 | 1.25 | 0.313626 | 3.21 | 0.005469 |
| MKP-1 | P28562 | S359 | -1.00 | 0.001034 | 2.51 | 0.010586 |
| SRF | P11831 | S77/79 | -1.87 | 0.198335 | 1.97 | 0.028672 |
| Fyn | P06241 | Y531 | -1.35 | 0.284851 | 1.81 | 0.056901 |
| caveolin-1 | Q2TNI1 | Y14 | -3.51 | 0.072589 | 1.74 | 0.043075 |
| p300 | Q09472 | S1834 | 1.92 | 0.002546 | 1.71 | 0.05587 |
| PPARG | P37231 | S112 | -2.41 | 0.06577 | 1.63 | 0.021592 |
| FRS2 | Q8WU20 | Y196 | -2.36 | 0.076686 | 1.62 | 0.024022 |
| MEK1 | Q02750 | S217 | -2.47 | 0.101358 | 1.50 | 0.013693 |
| VIM | P08670 | S39 | -1.82 | 0.270624 | 1.48 | 0.056439 |
| DAPK1 | P53355 | S308 | 2.19 | 0.007522 | 1.45 | 0.047372 |
| NGFR | P08138 | Y336 | -2.93 | 0.193009 | 1.37 | 0.016714 |
| APE1 | P27695 | S289 | 1.52 | 0.026386 | 1.28 | 0.063852 |
| 4E-BP1 | Q13541 | T46 | 1.77 | 0.001258 | -1.00 | 0.005399 |
| IkB-beta | Q15653 | S313/15 | -1.92 | 3.60E-04 | -1.22 | 0.033048 |
| EphA2 | P29317 | Y772 | -1.88 | 0.029456 | -1.22 | 0.075177 |
| FRS3 | Q8WU20 | Y416 | -4.37 | 0.025056 | -1.26 | 0.144433 |
| MKP-1 | P28562 | S296 | -8.08 | 0.040502 | -1.49 | 0.076952 |
| gp130 | P40189 | Y767 | -5.91 | 0.046097 | -1.60 | 0.002387 |
| ROCK2 | 075116 | S1134/7 | 1.20 | 0.001374 | -1.60 | 0.008068 |
| PKACa | P17612 | S338 | -1.64 | 5.46E-04 | -1.64 | 0.007801 |
| STAT2 | P52630 | Y690 | -1.05 | 0.185679 | -1.67 | 0.021569 |
| CREB | P16220 | S110/1 | -2.92 | 6.49E-04 | -1.68 | 0.010216 |
| Fas | P25445 | Y291 | -1.07 | 0.244763 | -1.74 | 0.022505 |
| Kit | P10721 | Y721 | -1.75 | 0.001468 | -1.75 | 0.008233 |
| Cystatin S | P01036 | S132 | -2.33 | 5.08E-04 | -1.78 | 0.003754 |
| Grb10 | Q13322 | S150 | -1.86 | 0.001074 | -1.86 | 0.009181 |
| Akt1 | P31749 | | -7.78 | 0.018698 | -1.87 | 0.171965 |
| Fos | P01100 | T232 | 1.51 | 0.020475 | -1.92 | 0.007536 |
| STAT6 | P42226 | Y641 | -1.94 | 0.001601 | -1.94 | 0.012476 |
| TAK1 | 043318 | T184 | -2.01 | 0.001519 | -2.01 | 0.006928 |
| NFAT4 | Q12968 | S163/5 | -2.22 | 0.001339 | -2.22 | 0.0083 |
| MEK5 | Q13163 | S311/15 | -6.20 | 0.009408 | -2.29 | 0.001913 |
| BLNK | Q8WV28 | Y96 | 1.14 | 0.009763 | -2.29 | 0.010163 |
| Smad3 | P84022 | S422/3/5 | -2.37 | 0.001995 | -2.37 | 0.008028 |
| p53 | P04637 | S6/9 | -2.43 | 0.001053 | -2.43 | 0.00479 |
| NFkB-p100 | Q00653 | S865 | -1.03 | 0.006376 | -2.54 | 0.008275 |
| CCR5 | P51681 | S336/7 | -2.62 | 0.001757 | -2.62 | 0.004969 |
| CaMK2-alpha | Q9UQM7 | T286 | -1.60 | 3.59E-04 | -3.12 | 0.006745 |
| SEK1 | P45985 | S80 | 1.00 | 0.083006 | -3.25 | 0.010964 |
| Akt1 | P31749 | S124/9 | -3.27 | 0.001009 | -3.27 | 0.011844 |
| Daxx | Q9UER7 | S668/71 | -3.45 | 0.00205 | -3.45 | 0.009993 |
| NFkB-p105 | P19838 | S337 | -3.16 | 0.180242 | -3.63 | 0.009452 |
| axin-1 | 015169 | S486 | -3.73 | 7.34E-04 | -3.73 | 0.003599 |
| Src | P12931 | S74 | -6.77 | 0.00113 | -3.78 | 0.005773 |
| CTNNB1 | P35222 | T41/45 | -4.12 | 9.09E-04 | -4.12 | 0.008661 |
| FLGE_ECOLI | Q8X8L1 | | -2.37 | 0.015468 | -4.18 | 0.004231 |
| FGFR1 | P11362 | Y154 | -2.32 | 0.103795 | -9.59 | 0.005387 |

Phophorylation of 21 peptides on the array were suppressed by two fold (p < 0.05) relative to un-stimulated cells in the presence of the cytokine IL-17 (Table 5). On stimulation with the cytokine IL-32, fifteen peptides on the array showed > 1.5-fold increase (p < 0.06) in phosphorylation relative to un-stimulated cells, representing the phosphorylation cellular targets for the cytokine IL-32, and phosphorylation of 18 peptides showed a more that two fold decrease (p ≤ 0.05) compared to un-stimulated cells (Table 5).

The results demonstrated that the overall pattern / trend of phosphorylation events in the presence of the cytokines IL-32 and IL-17 were largely different (Fig. 9), indicating overall differential kinase activities induced in the presence of these two cytokines. Differentially phosphorylated targets were defined as ≥ 1.5-fold increase (indicated by red bars) or ≥ 1.5-fold decrease (indicated by green bars) in phosphorylation compared to un-stimulated control cells (p<0.06). The differentially expressed candidates were submitted to the genesis software to generate a heatmap demonstrating the ratios of cytokine-stimulated differential phosphorylation compared to phosphorylation levels in un-stimulated control cells. From this analysis it was noted that cytokine IL-32 significantly induced the phosphorylation of TNF-receptor (TNF-R)-1 by more than four fold, while the state of TNF-R1 phosphorylation was not altered in the presence of IL-17 as compared to un-stimulated control cells (Table 4). Previous studies have hypothesized that IL-32-mediated pathogenesis in chronic inflammatory diseases may be in part dependent on TNF-α, for example as disclosed by Joosten et al. (2006, IL-32, a proinflammatory cytokine in rheumatoid arthritis. Proc Natl Acad Sci USA 103:3298-3303). However, the present disclosure is the first to demonstrate that IL-32-induced direct phosphorylation of TNF-R1.

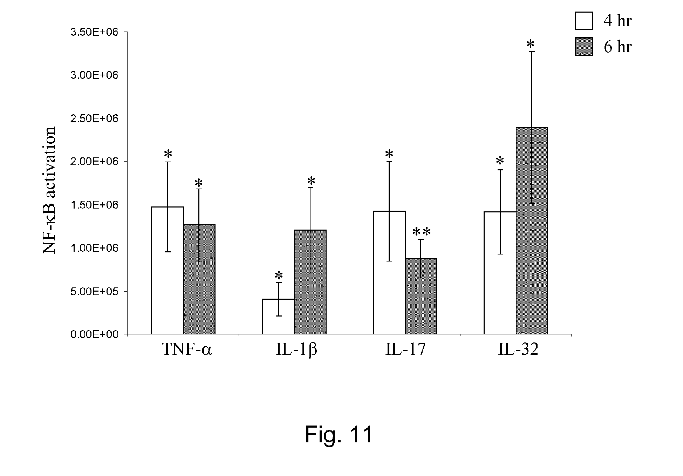

*Common downstream **responses** induced in the presence of cytokines IL-32 **and** IL-17:* The data disclosed herein conclusively demonstrated that both IL-32 and IL-17 induced the phosphorylation of two common proteins (Table 5 and Fig. 5). The induced downstream responses were monitored in the presence of IL-32 and IL-17 in macrophages and FLS cells. Both IL-32 and IL-17 induced significant ***(p*** < 0.05) gene expression of pro-inflammatory cytokines TNF-α, IL-23 and Gro-α, between 2 and 12-fold higher than un-stimulated control cells in macrophages (Fig. 10A). Similarly, a significant increase in TNF-α, IL-1β, IL-6 and IL-8 gene expression was observed in human FLS cells *(ex-vivo)* upon stimulation with either IL-17 or IL-32 (Fig. 10B). Furthermore both IL-32 and IL-17 induced direct activation of NF-κB in synovial fibroblasts (Fig. 11). Similarities in downstream responses upon stimulation with IL-32 and IL-17 supported the presence of common signalling intermediates for these cytokines.

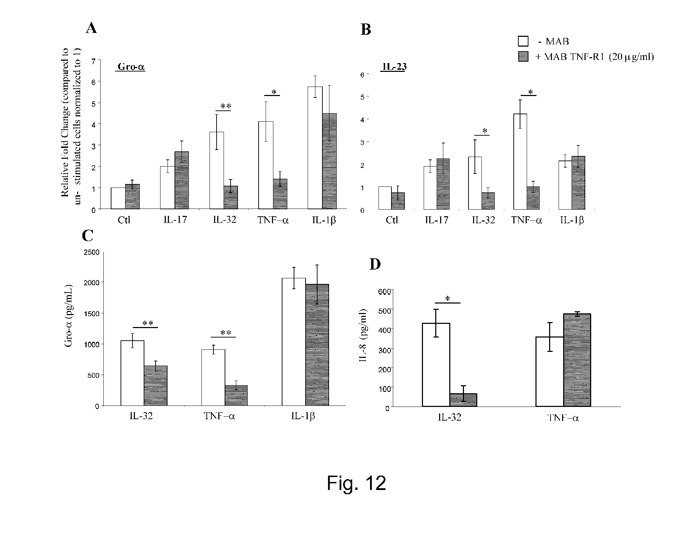

*Alteration of **IL-32-induced responses on blocking of TNF-receptor:*** Kinome analysis showed that IL-32 induced the phosphorylation of TNF-R1 (4.7-fold increase, p < 0.01), whereas the phosphorylation state of TNF-R1 after stimulation with IL-17 was not altered compared to un-stimulated cells (Table 5). Therefore we investigated the role of TNF-R1 on IL-32-induced downstream responses by blocking receptor activity using a specific neutralizing monoclonal antibody following the methods taught by Turner et al. (2007, Mechanism of TNFalpha-induced IL-1alpha, IL-1beta and IL-6 expression in human cardiac fibroblasts: effects of statins and thiazolidinediones. Cardiovasc. Re.s 76:81-90). Human macrophage-like THP-1 cells were pre-treated with monoclonal antibody MAB625 specific for TNF-R1 (20 µg/ml) for 1 hr, followed by stimulation with either IL-32 (20 ng/mL), IL-17 (20 ng/mL), TNF-α (10 ng/mL) or IL-1β (10 ng/mL) for 4 or 24 hr. Transcriptional responses were evaluated by qRT-PCR after 4 hr of stimulation. Gene expression of Gro-α (Fig. 12A) and IL-23 (Fig. 12B) induced in the presence of IL-32 and TNF-α were significantly (p < 0.05) suppressed by > 70%, in the presence of the neutralizing monoclonal antibody to TNF-R1. In contrast, transcriptional responses induced in the presence of either IL-17 or IL-1β was not altered (Fig. 12A and 12B). A similar trend was also observed at the protein level, blocking TNF-R1 significantly ***(p*** < 0.01) suppressed IL-32-induced chemokine Gro-α production by 35% ± 5% and TNF-α-induced Gro-αproduction by 60% after 24 hr of stimulation (Fig. 12C). There was no observed difference in IL-1β-induced Gro-α production (Fig. 12C). Similarly, blocking TNF-R1 significantly ***(p*** < 0.05) suppressed IL-32-induced IL-8 production by > 85% (Fig. 12D), but did not alter the production of TNF-α-induced IL-8 production (Fig. 12D).

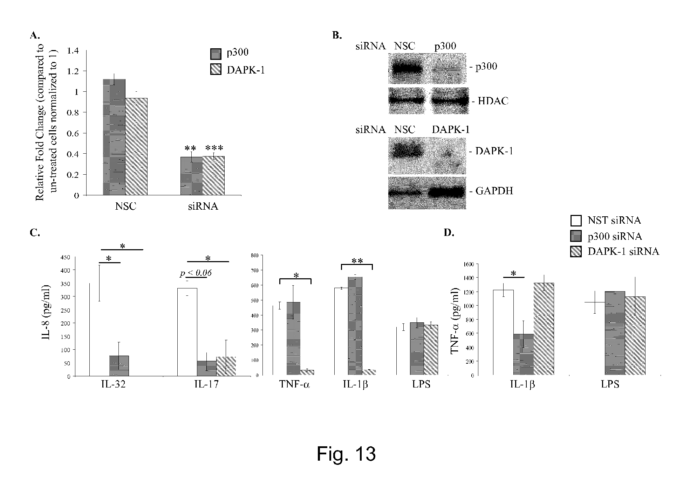

***Alteration** of IL-32- **and IL-17-induced** responses upon silencing of **p300 and DAPK-1 genes:*** To establish the functional relevance of the identified common targets, p300 and DAPK-1, we evaluated the impact of the knockdown of these proteins on cellular responses induced by IL-32 and IL-17, as well as those induced by pro-inflammatory cytokines TNF-α and IL-1β. Accell SMARTpool EP300 (p300) siRNA, DAPK-1 siRNA and a non-silencing control (NSC) siRNA were employed to knockdown the respective proteins in human macrophage-like THP-1 cells. Transcriptional analysis by qRT-PCR showed that gene expression for p300 and DAPK-1 were significantly ***(p*** < 0.01) suppressed by > 60% ± 5% on treatment with the respective siRNA, and remained unaltered in cells treated with NSC, when compared to controls cells not treated with siRNA (Fig. 13A). Immunoblots probing with specific antibodies revealed a significant knockdown of both p300 (EP300) and DAPK-1 proteins in cells treated with the respective siRNAs as compared to cells treated with NSC (Fig. 13B). Knockdown of protein expression was higher than that observed at the mRNA level (Fig. 13A and 13B). Knockdown of either p300 or DAPK-1 was not lethal to the cells as determined by monitoring LDH release for cellular cytoxicity (data not shown). Knockdown of p300 suppressed chemokine IL-8 production > 80% in the presence of either IL-32 or IL-17 (Fig. 13C). Knockdown of DAPK-1 abrogated the production of IL-8 upon stimulation with IL-32, and significantly (p < 0.05) suppressed IL-17-induced IL-8 production by > 70% (Fig. 8A). We have demonstrated that IL-32 can induce both IL-8 message (Fig. 13A) and protein production (Fig. 7C) in macrophage-like THP-1 cells. Even though IL-17 did not significantly enhance IL-8 gene expression (Fig. 8A), IL-8 protein production was significantly induced upon stimulation with IL-17 (Fig. 7C) in macrophage-like THP-1 cells. Taken together, it was valid to employ IL-8 protein production as a read-out for assessing the impact of p300 and DAPK-1 knockdown on IL-32 and IL-17-induced downstream responses. In addition, knockdown of DAPK-1 significantly suppressed the production of IL-8 by > 90% upon stimulation with pro-inflammatory cytokines TNF-α and IL-1β (Fig. 13C). Similarly, knockdown of p300 significantly ***(p*** < 0.05) suppressed IL-1β-induced production of TNF-α by > 50% (Fig. 13D). In contrast, knockdown of either p300 or DAPK-1 did not alter LPS-induced responses in macrophage-like THP-1 cells (Fig. 13C and 13D).

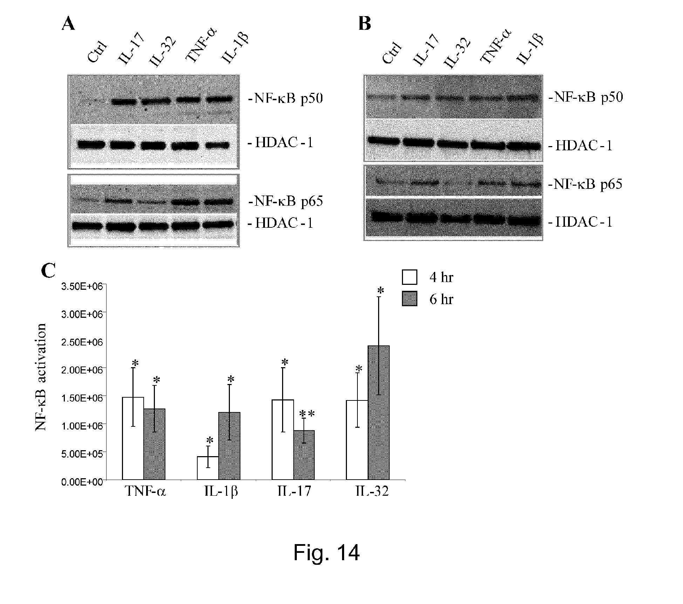

*Activation of transcription factor **NF-κB** in the presence of IL-32 **and** IL-17:* This study conclusively demonstrated that the cytokine IL-32 and IL-17 phosphorylated common targets, p300 and DAPK1, in human macrophages. The presence of common targets in macrophages was supported by the observation that both IL-32 and IL-17 induced similar level of IL-23 mRNA expression (Fig. 8A) and chemokine IL-8 production in human macrophage-like THP-1 cells (Fig. 7C). In this study it was also observed that both the cytokines IL-32 and IL-17 induced similar levels of TNF-a mRNA expression in human FLS cells (Fig. 8B). Therefore to further evaluate similarity of induced downstream response in synovial fibroblasts, this study analyzed the direct activation of transcription factor NF-κB. Human FLS cells or macrophage-like THP-1 cells were stimulated with either IL-32 (20 ng/mL), IL-17 (20 ng/mL), TNF-α (10 ng/mL) or IL-1β (10 ng/mL) for 30 mins. Equivalent loading of nuclear extracts (5-8 µg) were probed in immunoblots with antibodies specific to either NF-κB subunits p50, p65 or HDAC-1 as input control. Both IL-17 and IL-32 mediated nuclear translocation of NF-κB p50 in both FLS cells (Fig. 14A) and macrophage-like THP-1 cells (Fig. 14B), the levels were comparable to that seen with known activators of NF-κB i.e. pro-inflammatory cytokines TNF-α and IL-1β (Fig. 14A and 14B). Nuclear translocation of NF-κB subunit p65 was mediated in the presence of IL-17 in both FLS cells (Fig. 14A) and macrophages (Fig. 14B), and in contrast IL-32 had limited effect of the nuclear translocation of p65 (Fig. 14A and 14B). We also monitored the activity of NF-κB using a luminescence assay in-vitro employing rabbit synovial fibroblast cell line (HIG-82) in the presence of IL-32 and IL-17. The activation of NF-κB was monitored after 4 and 6 hr of treatment. The critical inflammatory transcription factor NF-κB was activated by both, cytokine IL-32 and IL-17, in the synovial fibroblasts (Fig. 14C). Taken together, these results demonstrated that both IL-17 and IL-32 can activate the transcription factor NF-κB, but this may be regulated by dimerization of different NF-κB subunits for the two cytokines. This is consistent with our hypothesis that these two cytokines have common downstream cellular effects and protein targets, but these are regulated by differential signalling mechanisms.

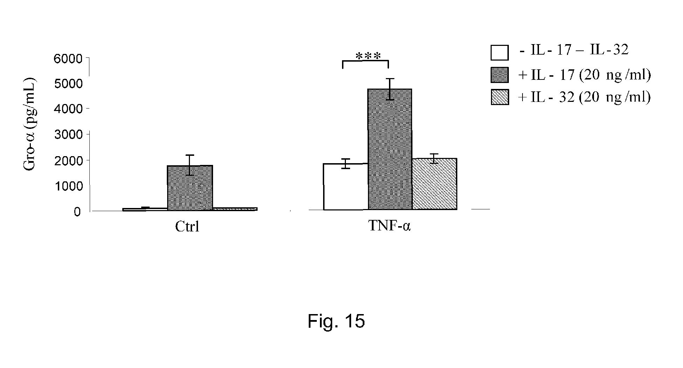

***Differential modulation of TNF-α-induced** chemokine production in the presence of IL-32 **and IL-17:*** Inflammatory responses do not function as a linear cascade of events. It is a complex network of cross-talk between different cellular inflammatory mediators and signalling cascades. The kinome analysis revealed that the phosphorylation profiles induced by IL-32 and IL-17 were largely different. Therefore in order to establish that differential regulatory processes were employed by these two cytokines, this study investigated IL-32 and IL-17-induced downstream response in the presence of pro-inflammatory cytokines TNF-a and IL-1β. In this study we evaluated the impact of IL-32 and IL-17-induced responses in the presence of the acute inflammatory mediators, TNF-α and IL-1β. Macrophage-like THP-1 cells and human FLS cells were stimulated with either TNF-α (10 ng/mL) or IL-1β (10 ng/mL) in the presence and absence of either IL-32 or IL-17 (20 ng/mL). The tissue culture supernatants were monitored for the production of chemokines Gro-αand IL-8 by ELISA using specific antibodies. There were no significant alteration of IL-β-induced chemokine production in either macrophages or FLS cells in the presence of either IL-32 or IL-17 (data not shown). Similarly, no synergistic enhancement of TNF-α-induced chemokine production was observed in the presence either IL-32 or IL-17 in macrophage-like THP-1 cells (data not shown). In contrast, TNF-α-induced chemokine Gro-αproduction was significantly (p < 0.001) and synergistically enhanced in the presence of IL-17 in human FLS cells after 24 hr of stimulation, but this synergistic effect was not observed in presence of the cytokine IL-32 (Fig. 15). These results indicated that TNF-α-induced chemokine responses were differentially modulated in the presence of cytokines IL-32 and IL-17 in human FLS cells.

Modulation of IL-32 and IL-17-mediated cellular responses by innate immune effector cationic peptide LL-37: No studies to date have explored the effects of innate immune defence peptide LL-37 on chronic inflammatory cytokine induced cellular responses.

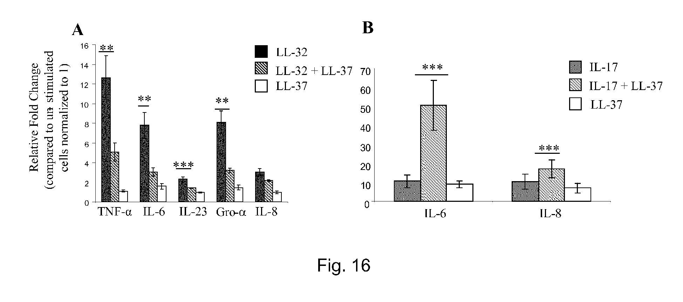

In this study we investigated the impact of the human cathelicidin peptide LL-37 on cytokines IL-32- and IL-17-induced cellular responses. Cytokine IL-17-induced expressions of pro-inflammatory genes or chemokine gene expression were not altered in the presence of the peptide LL-37 in human macrophages (data not shown). Whereas IL-32-induced gene expression for TNF-α, IL-6 and Gro-α(CXCL1) were all significantly (p < 0.01) suppressed by more than 50%, and the expressions of IL-23, IL-8 (CXCL8) and NF-□B1A were suppressed between 30% - 40%, in the presence of LL-37 in human macrophage-like THP-1 cells (Fig. 16A). In contrast, some of the IL-17-induced transcriptional responses were significantly enhanced by the presence of the peptide LL-37 in human FLS cells (Fig. 16B). The differential trends of alteration of cellular responses on stimulation with IL-32 or IL-17, in the presence of other molecules known to be elevated during inflammation such as cytokine TNF-α (Fig. 15) or human cathelicidin peptide LL-37 (Fig. 16) supported the kinome analysis, taken together indicating that the cytokines IL-32 and IL-17 may be engaging different regulatory cellular processes.

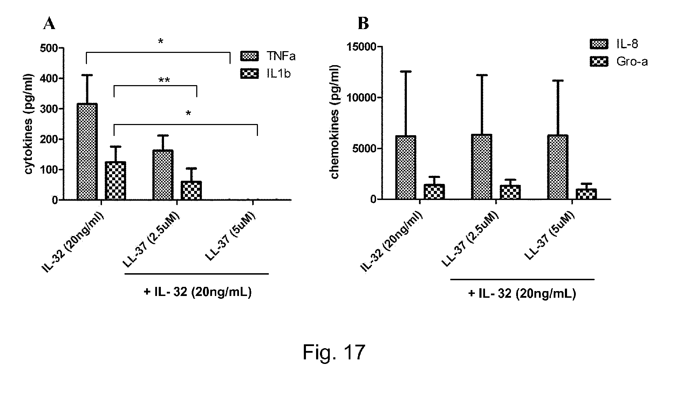

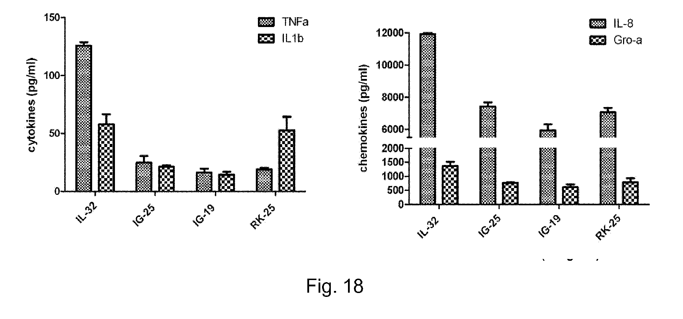

Impact of cationic peptide LL-37 and its synthetic derivatives on IL-32-induced protein production: Human macrophage-like THP-1 cells were stimulated with IL-32 (20 ng/mL) in the presence and absence of cationic peptide LL-37, tissues culture supernatants were monitored for the production of pro-inflammatory cytokines TNF-α and IL-1β, and chemokines after 24 hr of stimulation. LL-37 significantly suppressed IL-32-induced production of both TNF-α and IL-1β (Fig. 17A), but chemokine production which is required for anti-infective immunity was not altered by LL-37 (Fig. 17B). As LL-37 suppressed IL-32-mediated pro-inflammatory responses, we also evaluated the impact of LL-37 derived synthetic peptides. LL-37-derived synthetic peptides, in particular IG-25 and IG-19, significantly suppressed IL-32-induced pro-inflammatory responses (TNF-α and IL-1β) production, and similar to the parent peptide did not completely neutralize chemokine responses (Fig. 18). These results were consistent with the paradigm of 'selective' anti-inflammatory mechanism of cationic host defence peptides and their synthetic derivatives in the context of pathogen-mediated sepsis as previously described. No studies to data have explored the impact of these peptides in the context of chronic inflammation. Our results showed that chronic inflammatory cytokine IL-32-induced pro-inflammatory protein production i.e. TNF-α and IL-1β were significantly and 'selectively' suppressed by human cathelicidin peptide LL-37 and its derivative peptides. This provides the data that supports further investigation of these peptide candidates as potential therapeutics for diseases characterized by chronic inflammation.

### Discussion

Immune-mediated chronic inflammatory diseases such as RA result from a dynamic and complex interplay of regulatory networks of signaling pathways. The complexity of these interactions can limit the efficacy of targeting a single molecule or one specific pathway for the pharmacologic management of these disorders. For example, in RA many patients do not respond to biologic therapies that target the TNF-mediated pathway and these therapeutics do not completely control the progression of the disease. This suggests that despite the central role proposed for TNF-α, there exist a complex network of various cytokine-mediated regulatory pathways contributing to the inflammatory microenvironment. The identification of overlapping nodes within the different cytokine-mediated networks could be valuable as potential drug targets for diseases characterized by chronic inflammation.

This study investigated the molecular mechanisms induced by two new cytokines IL-32 and IL-17. These cytokines are emerging as critical contributors in the pathogenesis of various chronic inflammatory and autoimmune diseases. IL-32 is a potent pro-inflammatory cytokine. Elevated levels of IL-32 have been directly correlated to severity of chronic inflammatory disorders including RA and IBD. However, the network of regulatory signalling pathways induced by IL-32 has yet to be completely defined. In contrast, molecular regulation mediated by IL-17 has been relatively well characterized. IL-17 is known to be a potent mediator of pro-inflammatory cytokines, chemokines, acute phase response elements and defensins. IL-17 is associated with the pathophysiology of autoimmune diseases including RA, IBD, systemic lupus, psoriasis and autoimmune encephalitis. Recently, this cytokine was also shown to be expressed in atherosclerotic plaques. Despite similarities in pro-inflammatory functions of IL-32 and IL-17, and both being described as therapeutic cytokine targets, previous reports have speculated that responses mediated by these cytokines may be differentially dependent on the TNF pathway. The role of IL-32 in mediating influx of inflammatory cells and subsequent cartilage damage in arthritis is dependent on TNF-related mechanisms, and in contrast IL-17-mediated inflammation under arthritic conditions is in part TNF-independent. To date, there are no studies that provide insight into the differential regulatory processes or explain the basis for the differential TNF-pathway-dependence in IL-32- and IL-17-mediated inflammatory responses. The key findings in this study were; (i) IL-32-mediated responses, but not IL-17, were dependent on TNF-R1 (Table 4 and Fig. 12), and (ii) p300 and DAPK-1 were common signaling intermediates for both IL-32 and IL-17 (Table 4 and Fig. 5), and that knockdown of these proteins impaired cytokine-mediated downstream responses (Fig. 13).

The present disclosure demonstrates that unlike IL-17, cytokine IL-32-mediated downstream cellular responses are dependent on TNF-R1 (Fig. 12), which supported the kinome analysis (Table 4). TNF-α stimulation was used as a positive control for the TNF-R1 neutralization assays. As expected, blocking of TNF-R1 suppressed TNF-α-induced transcriptional responses and Gro-αproduction, but did not inhibit IL-8 production (Fig. 12D). Two receptors with distinct signalling mechanisms have been defined for the pro-inflammatory events induced in the presence of the cytokine TNF-α, TNF-R1 (TNFRSF1A or p55) and TNF-R2 (TNFRSF1B or p75). TNF-α-induced IL-8 production up on blocking TNF-R1 indicates that this response is probably mediated by the TNF-R2 signalling pathway. These results also suggest that IL-32-induced responses are not a result of TNF-α feedback loop, and may be directly dependent on TNF-R1 activity. Therefore, these data indicate that the TNF-pathway-dependent role of IL-32 in chronic inflammation is due to the engagement of the cytokine with TNF-R1. The only direct interacting protein partner demonstrated for IL-32 is a neutrophil-derived serine protease, proteinase 3. No other receptor has been described for IL-32 to date. However, this is the first study to provide molecular insight for the speculations that inflammatory functions of IL-32 are in part dependent on the TNF-pathway.

p300 and DAPK-1 are identified as common protein phosphorylation targets for IL-32 and IL-17. The data disclosed herein indicate that there is overlap in the inflammatory networks induced by these two cytokines, and commonalities in the regulatory processes triggered by these cytokines. The functional relevance of the identified common protein targets was confirmed using knockdown studies, which showed that knockdown of either p300 or DAPK-1 altered both IL-32- and IL-17-induced downstream responses (Fig 13C). In addition, knockdown of p300 and DAPK-1 also altered certain TNF-induced and IL-1β-induced cellular responses (Fig. 13C and 13D). These results were consistent with the hypothesis that common protein targets of IL-32 and IL-17 would likely be involved in TNF-dependent and independent cellular responses.

The transcriptional co-activator p300, in concert with other transcription factors, triggers gene expression in the presence of inflammatory stimuli. For example, p300 is essential for IL-1β-induced prostaglandin release, and phospholipase A(2) and NF-κB activation in human tracheal smooth muscles. The data disclosed herein demonstrate that IL-32 and IL-17 stimulation resulted in the phosphorylation of p300 at Ser-1834 (Table 4 and Fig. 5). A previous report has demonstrated that phosphorylation of p300 at Ser-1834 results from the nuclear translocation of AKT upon the activation of the PI3K/AKT pathway in response to pro-inflammatory cytokine TNF-α. Likewise the involvement of activation of AKT pathway upon stimulation with IL-32 has been recently demonstrated in osteoclasts, and for IL-17 in retinal astrocytes and bronchial epithelial cells. Taken together, this demonstrates that the cytokines IL-32 and IL-17 induce the phosphorylation of p300 at Ser-1834 by engaging the PI3K/AKT pathway, resulting in the activation of NF-κB.

The second protein target identified in this study for cytokines IL-32 and IL-17 was DAPK-1, which is an apoptotic regulator and has been implicated in the disease process of several cancers. IL-32 is known to promote apoptosis of keratinocytes in atopic dermatitis, which supports our results that identified an apoptosis regulator, DAPK-1, as a protein target for IL-32. Another study has shown that inflammation can lead to aberrant DNA methylation of the DAPK-1 gene in a model of IBD. In addition, DAPK has been recently described to be a potential therapeutic target for chronic neurodegenerative diseases. Since dysregulation of inflammation has been correlated with several cancers as well as with neurodegenerative disorder such as Alzheimer's disease, it can be assumed that DAPK-1 plays a regulatory role in pathways induced in the presence of chronic inflammatory stimuli such as the cytokines investigated in this study.

The key findings are as follows. Two proteins i.e. p300 and DAPK-1 have been identified as targets for the cytokines IL-32 and IL-17. These protein targets i.e. p300 and DAPK-1, may represent overlapping nodes of different signaling pathways, which may or may not be TNF-dependent, thereby contributing to chronic inflammatory diseases. We also identified five peptides i.e., DAPK1, p300, 4E-BP1, APE1, and Fos, that are unique phosphorylation targets for chronic inflammatory cytokines IL-32 and IL-17. These peptides are useful as therapeutic targets for immune-mediated chronic inflammatory diseases. Evidence is disclosed that demonstrates cytokines IL-32- and IL-17-mediated inflammatory responses are differentially regulated, and that these may be TNF-pathway-dependent or TNF-pathway-independent respectively. We have identified TNF-R1 as a phosphorylation target for IL-32 and have demonstrated that IL-32-induced responses are dependent on TNF-R1 activity. This provided molecular insight into TNF-pathway dependent mechanism of IL-32 in inflammation. This study demonstrated that cationic host defence peptide LL-37 and its synthetic derivative peptides can significantly suppress IL-32-induced inflammatory responses such as production of critical pro-inflammatory mediators TNF-α and IL-1β. This opens up avenue for further investigation of these cationic peptides for exploring their potential in IL-32-mediated inflammation critical in chronic inflammatory and autoimmune diseases. This warrants further investigation into the potential clinical application of these peptides for a wide range of diseases characterized by chronic inflammation which includes RA, IBD, COPD, atherosclerosis, and certain cancers. The protein targets identified for cytokines IL-32 and IL-17 in this study can be employed to screen small molecules (such as cationic peptides) as potential anti-inflammatory agents.

### Example 3: Use of L-azidohomoalanine for selective enrichment of proteins produced in the presence of pro-inflammatory cytokines TNF-α and IL-1β

Molecular indicators of a disease state or activity (biomarkers) can provide insight into the pathogenesis, new therapeutic targets, and molecules that may be used to predict the responsiveness of candidate therapeutics. Identification of such molecular indicators is an important goal in biomedical research, including for diseases characterized by chronic inflammation. Gene-expression monitoring (GEM) or cytokine profiling from serum or other body fluids has been used in recent years to define biomarkers for systemic inflammation (Criswell, 2010, Gene discovery in rheumatoid arthritis highlights the CD40/NF-kappaB signalling pathway in disease pathogenesis. Immunol. Rev. 233:55-61). However, GEM is not a robust indicator for the phenotype of the disease, and multianalyte cytokine profiling are hypothesis-driven surrogate endpoints that does not differentiate between different systemic inflammatory disorders (O'Hara et al., 2006, Cell-surface and cytokine biomarkers in autoimmune and inflammatory diseases. Drug Discov. Today 11:342-347). Also, global proteomic approaches such as 2D gel electrophoresis or with isotope labelling, do not differentiate between newly synthesized (nascent) proteins in response to a stimulus and those from the pre-existing pools, since they are chemically identical.

In this study we used an unnatural amino acid L-azidohomoalanine (AHA; an analogue of methionine) to selectively enrich nascent proteins synthesized in response to pro-inflammatory cytokines TNF-α and IL-1β, against a background of pre-existing proteins in the cellular environment. Metabolic labelling with AHA provides nascent proteins with azide-bearing functional group, thus making these molecules distinct from the pool of pre-existing cellular proteins. A biotin alkyne reagent can be covalently coupled to the reactive azide group of the AHA-modified proteins, and subsequently used for enrichment of the nascent proteins by affinity purification.

We labelled three different cell types with AHA: (i) human monocytic THP-1 cell line, (ii) human T-cell Jurkat cell line, and (iii) a rabbit synovial fibroblast HIG-82 cell line. AHA labelling was cytotoxic to the synovial fibroblast cell line evidenced by release of lactate dehydrogenase (LDH) into tissue culture supernatants (data not shown) within 4 hr. However, AHA was not cytotoxic to human T-cell Jurkat cells or to the human monocytic THP-1 cells. Both THP-1 cells and Jurkat cells became plastic adherent after culturing in serum-free, methionine-free media for 60 min prior to labelling with AHA. Human monocytic THP-1 cells were further used in this study for metabolic labelling with AHA followed by quantitative proteomics using ITRAQ^{®} reagents (ITRAQ is a registered trademark of Ab Sciex PTE. Ltd., Singapore, Singapore) in order to identify nascent proteins induced by inflammatory cytokines.

Human monocytic THP-1 (ATCC^{®} TIB-202) cells and Jurkat T-cell line (ATCC^{®} TIB-152) were cultured in RPMI-1640 media containing 2 mM L-glutamine and 1 mM sodium pyruvate, supplemented with 10% (v/v) FBS, and maintained in a humidified incubator at 37°C and 5% CO₂. Where indicated the THP-1 cells were differentiated to plastic-adherent macrophage-like cells by treatment with phorbol 12-myristate 13-acetate (PMA; Sigma-Aldrich Canada, Oakville ON) as taught by Mookherjee et al. (2006). A rabbit synoviocyte cell line HIG-82 (ATCC^{®} CRL-1832) was cultured in Ham's F-12 growth medium containing glutamine supplemented with sodium pyruvate (referred to as complete F-12 media henceforth), containing 10% (v/v) FBS in a humidified incubator at 37°C and 5% CO₂. Confluent HIG-82 cells were trypsinized with 1:3 dilution of 0.5% trypsin-EDTA in Hanks' balanced salt solution. Cellular cytotoxicity was evaluated after AHA labelling and upon stimulation with the various cytokines for all the cell types used in this study by monitoring the release of lactate dehydrogenase (LDH) employing a colorimetric detectionkit (Roche Diagnostics, Laval, QC, Canada). Recombinant human cytokines TNF-α and IL-1β were obtained from eBioscience, Inc (San Diego, CA, USA).

***ELISA** immunoassay:* Tissue culture (TC) supernatants were centrifuged at 1500 X g for 7 min to obtain cell-free samples, aliquoted and stored at -20°C until further use. Production of IL-8 was monitored using specific antibody pairs for ELISA (R&D Systems, Inc. Minneapolis, MN, USA), as per the manufacturer's instructions. The concentration of IL-8 in the TC supernatants was evaluated by establishing a standard curve with serial dilutions of the recombinant human IL-8 as required.

*AHA labelling, **biotin tagging** of nascent proteins, **and affinity purification:*** Cells were washed with warm D-PBS, and cultured in methionine-free, serum-free RPMI media at 37°C 5% CO₂ for 60 min. The cells were then incubated in 100 µM CLICK-IT^{®} AHA (Invitrogen Canada Inc.)(CLICK-IT is a registered trademark of Molecular Probes Inc., Eugene, OR, USA) in methionine-free and serum-free RPMI media in the presence and absence of either TNF-α (10 ng/ml) or IL-1β (10 ng/ml) for 4 hr. TC supernatants were monitored for the production of IL-8 by ELISA. Metabolic labelling of the cells with AHA reagent in the presence of the cytokine stimulants would result in induced nascent proteins with azide-bearing functional group, thus making them distinct from pool of pre-existing cellular proteins. Also these functional groups were further used to tag the nascent proteins with a biotin-alkyne reagent as follows. The cells were washed with D-PBS, followed by preparing cell lysates in lysis buffer containing 50 mM Tris-HCl, 1% SDS, 250 U/ml of benzonase nuclease and protease and phosphatase inhibitor cocktails (Sigma-Aldrich Canada Inc.). Total protein was estimated in the cell lysates using micro BCA analysis. Equivalent amount of total protein from each sample was acetone precipitated and then treated with CLICK-IT^{®} Biotin-alkyne reagent (Invitrogen Canada Inc.) per the manufacturer's instructions. The biotin-alkyne reagent thus covalently coupled to the reactive azide group of the AHA-modified proteins was used to subsequently enrich the nascent proteins by affinity purification using ULTRALINK^{®} Immobilized NEUTRAVIDIN^{®} resin (ULTRALINK and NEUTRAVIDIN are registered trademarks of Pierce Biotechnology Inc, Rockford, IL, USA), and the bound proteins were eluted using 6 M guanidinium hydrochloride.

*Immunoblots:* Eluates obtained from affinity purification were electrophoretically resolved on 4% - 12% NUPAGE^{®} Bis-Tris gels (Invitrogen Canada Inc.), followed by transfer to nitrocellulose membranes (Millipore). The membranes were subsequently blocked with TBST (20 mM Tris pH 7.5, 150 mM NaCl, 0.1% Tween 20) containing 5% skimmed milk powder, and probed with HRP-linked anti-biotin antibody (Cell Signaling Technology Inc., in TBST containing 3% skimmed milk powder. The membranes were developed with the Amersham ECL detection system (GE Healthcare, Baie d'Urfe QC, Canada).

***Quantitative proteomics employing isobaric tag** for relative **and absolute quantitation (ITRAQ^{®}):*** Amine-modifying ITRAQ^{®} reagents multiplex kit (Applied Biosystems, Foster City, CA, USA) was employed for relative quantitation of purified nascent proteins. Affinity purified eluates were acetone precipitated at -20°C overnight. Proteins were dissolved in 20 µl of ITRAQ^{®} dissolution buffer (Applied Biosystems) and further processed as per the manufacturer's instructions. Briefly, proteins were reduced and the cysteines blocked using the reagents in kit, followed by digestion of the protein samples with provided trypsin solution overnight at 37 °C. The trypsin-digested protein samples were labelled with the ITRAQ^{®} isobaric tags as follows: Eluates un-stimulated (control) samples was labelled with ITRAQ^{®} isobaric tag 115, TNF-α-stimulated samples with tag 116, and IL-1β-stimulated sample with isobaric tag 117. The contents from each of the ITRAQ^{®} reagent-labelled sample was combined together and processed for nanoflow liquid chromatography coupled to tandem mass spectrometry (LC-MS/MS).

***Quantitative real-time** PCR **(qRT-PCR):*** RNA was isolated using the QIAGEN^{®} RNEASY^{®} kit as per the manufacturer's instructions (QIAGEN and RNEASY are registered trademarks of Qiagen GmbH, Hilden, Fed. Rep. Germany). Gene expression was subsequently analyzed by qRT-PCR using SUPERSCRIPT^{®} III Platinum Two-Step qRT-PCR Kit (SUPERSCRIPT is a registered trademark of Invitrogen Corp., Carlsbad, CA, USA) with SYBR^{®} Green (SYBR is a registered trademark of Molecular Probes Inc., Eugene, OR, USA), according to the manufacturer's instructions, in the ABI PRISM^{®} 7300 sequence detection system (ABI PRISM is a registered trademark of Applera Corp., Foster City, CA, USA). Fold changes were calculated by the comparative Ct method as taught by Pfaffl (2001) after normalization with 18sRNA.

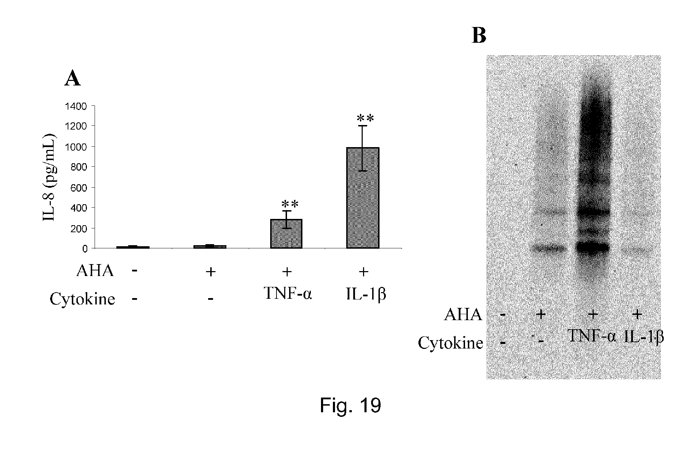

AHA-labelled human monocytic THP-1 cells were stimulated with either IL-1β (10 ng/ml) or TNF-α (10 ng/ml) for 4 hr. Cytokine treatment of AHA-labelled THP-1 cells was not cytotoxic as evaluated by monitoring LDH release in the tissue culture (TC) supernatant (data not shown). TC supernatants were also monitored for IL-8 production by ELISA. Both TNF-α and IL-1β showed significant *(p* < 0.001) production of IL-8 after 4 hr of stimulation (Fig. 19A). IL-8 production was more than 2-fold greater upon IL-1β stimulation when compared to cells stimulated with TNF-α (Fig. 19A). The cell lysates were further treated with a biotin alkyne reagent for tagging biotin onto the azide reactive group of the AHA-containing nascent proteins. The alkyne-biotin tagged AHA-containing nascent proteins were enriched by affinity purification using Neutravidin resin and the eluates were probed with anti-biotin antibody using Western blots. IL-1β- and TNF-α-treated samples showed increased amounts of biotinylated proteins compared to un-stimulated control cells (Fig. 19B). The eluates obtained from samples treated with IL-1β appeared to have greater amount of biotinylated enriched proteins compared to samples treated with TNF-α (Fig. 19B), which was consistent with the trend of protein production seen upon monitoring cytokine-induced IL-8 production by ELISA (Fig. 19A).

The AHA-containing nascent proteins enriched by affinity purification were quantitated using isobaric ITRAQ^{®} quantitative proteomics reagents. This was done to identify and estimative the relative abundance of nascent proteins enriched in the cytokine-treated samples compared to un-stimulated cells. Protein eluates after affinity purification were labelled with isobaric ITRAQ^{®} reagents; tag 115 for un-stimulated control cells, tag 116 for TNF-α-treated cells, and tag 117 for cells treated with IL-1β. Samples from four independent experiments were individually examined by LC-MS/MS. Peptides identified with 95% confidence were selected for further analysis. The mass spectrometry data was analyzed using Global Proteome Machine (GPM-http://www.thegpm.org/). We identified a total of 2,440 proteins from the four independent experiments, of which 1,449 proteins were identified in at least two out of the four replicates. Proteins were defined to be induced only if the relative abundance was more than 1.5-fold greater than un-stimulated cells, and if this increase was statistically significant *(p* < 0.05) across four independent biological replicates. Based on these selection criteria, we identified 16 candidates as nascent proteins that were induced (≥ 1.5-fold, *p* < 0.05) upon stimulation with either TNF-α or IL-1β or both after 4 hr of cytokine stimulation (Table 6).

**Table 6:**

| | TNF-α | | IL-1β | |
|---|---|---|---|---|
| | Fold Change | *p*≤ | Fold Change | *p*≤ |
| DDX17 - DEAD (Asp-Glu-Ala-Asp) box polypeptide 17 | 1.85 | 0.03 | 2.2 | 0.001 |
| CAP1 - CAP, adenylate cyclase-associated protein 1 | 1.76 | 0.06 | 1.9 | 0.009 |
| ALDOA - aldolase A, fructose-bisphosphate | 1.59 | 0.04 | 1.5 | 0.05 |
| PYGL - phosphorylase, glycogen, liver | 1.63 | 0.05 | 1.7 | 0.05 |
| CTPS - CTP synthase | 1.58 | 0.05 | 1.5 | 0.06 |
| HMGN1 - high-mobility group nucleosome binding domain 1 | 1.87 | 0.05 | 1.5 | 0.08 |
| EIFA3 - eukaryotic translation initiation factor 3, subunit A | 1.59 | 0.04 | 1.2 | 0.09 |
| PRTN3 - proteinase 3 | 1.52 | 0.03 | 0.8 | NS |
| HSPA4 - heat shock 70kDa protein 4 | 1.56 | 0.03 | 1.1 | NS |
| AIFM1 - apoptosis-inducing factor, mitochondrion-associated, 1 | 1.59 | 0.04 | 1.2 | NS |
| CALR - calreticulin | 1.51 | 0.05 | 1.0 | NS |
| VDAC3 - voltage-dependent anion channel 3 | 1.51 | 0.05 | 1.1 | NS |
| RAB14 - member RAS oncogene family | 1.53 | 0.05 | 1.2 | NS |
| PSME2 - proteasome (prosome, macropain) activator subunit 2 (PA28 beta) | 2.16 | 0.08 | 2.3 | 0.04 |
| SEC22B - vesicle trafficking protein homolog B | 1.24 | NS | 1.6 | 0.01 |
| LCP1 - lymphocyte cytosolic protein 1 (L-plastin) | 1.08 | NS | 1.5 | 0.03 |

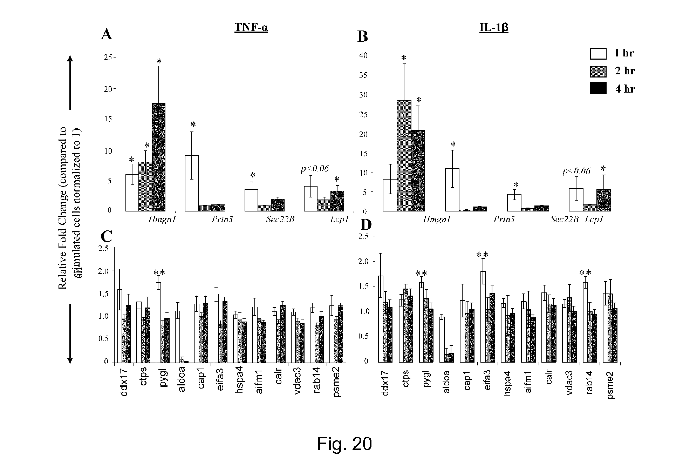

***TNF***-***α and IL-1β induced** gene **expression** of **five** common **identified** molecular **indicators:*** In order to confirm that the identified 16 proteins (Table 6) were indeed being induced upon stimulation with the cytokines, we evaluated kinetics of cytokine-induced gene expression for these candidates by quantitative real-time PCR (qRT-PCR). Human THP-1 monocytic cells were stimulated with either TNF-α or IL-1β, and transcriptional responses for the 16 identified candidates (Table 6) were monitored after 1, 2 and 4 hr of cytokine stimulation. Both TNF-α and IL-1β induced gene expression of five of the 16 candidates (Fig. 20). Gene expression of HMGN1, LCP-1, sec22B and prtn3 (C-ANCA), was up-regulated between 4 and 18-fold (*p* ≤ 0.05), in the presence of either TNF-α or IL-1β after 1 hr of stimulation (Figs. 20A and 20B). Both TNF-α and IL-1β induced a modest (≥ 1.5-fold) but significant (*p* ≤ 0.01) up-regulation of a glycogen phosphorylase enzyme, PYGL (Figs. 20C and 20D). TNF-α did not uniquely induce the gene expression of any candidate monitored in this study. In contrast, there was a modest (≥ 1.5-fold, *p* ≤ 0.01) up-regulation of RAB 14 and Eifa3, upon stimulation with IL-1β but not TNF-α (Fig. 20D).

Induction of HMGN1 nascent protein (Table 6) as well as up-regulation of gene expression (Fig. 20) was demonstrated upon stimulation with either TNF-α or IL-1β in this study. HMGN1 is a nucleosome-binding protein and is an emerging factor in transcriptional regulation of proto-oncogenes and tumour suppressor genes by affecting histone modifications (Gerlitz, 2009, DNA repair and cancer. Biochim. Biophys. Acta 1799:80-85). Post-translational modification of HMGN1 which includes phosphorylation and acetylation, affects the interaction of HMGN1 with its chromatin targets and is thought to regulate cellular responses to environmental stimuli (Zhang et al., 2009, HMG modifications and nuclear function. Biochim. Biophys. Acta 1799:28-36). However the role of HMGN1 in inflammation has not yet been defined. This is the first study that demonstrates that HMGN1 is significantly induced upon stimulation with either TNF-α or IL-1β in human monocytic cells.

Nascent protein synthesis of prtn3 (C-ANCA antigen) was demonstrated to be significant (*p* ≤ 0.05) upon TNF-α stimulation but not with IL-1β stimulation (Table 6). However, upon monitoring kinetics of gene expression of prtn3, we showed significant up-regulation of prtn3 mRNA after 1 hr of stimulation with both TNF-α and IL-1β (Figs. 20A and 20B). In this study, even though *de novo* protein synthesis of LCP-1 and sec22B was significantly demonstrated in the presence of IL-1β but not TNF-α (Table 6), monitoring of gene expression showed that both LCP-1 and sec22B mRNA was up-regulated after 1 hr of stimulation with either TNF-α or IL-1β (Figs. 20A and 20B). LCP-1 also known as L-plastin, is a leukocyte-specific protein, and it has been previously demonstrated that various inflammatory stimuli such as chemokines, bacterial lipopolysaccharide and immune complexes induce phosphorylation of LCP-1, which is required for integrin-mediated adhesion of leukocytes. However, association of the vesicular protein sec22B has not been defined with inflammatory process to date.

Further, we quantitated the enriched AHA-labelled nascent proteins using isobaric tags i.e. ITRAQ^{®} reagents by quantitative proteomics. No studies to date have used this technology to define nascent proteins upon cytokine stimulation. Using a combination of metabolic labelling of nascent proteins by AHA, followed by quantitative proteomics, we identified a panel of 16 nascent proteins to be induced upon stimulation with either TNF-α or IL-1β. We further confirmed that gene expression of five of the 16 identified candidates were significantly (*p* ≤ 0.05) up-regulated upon stimulation with either TNF-α or IL-1β. This is the first study to demonstrate a methodology that can be used to quantitatively define cytokine-induced nascent proteins, which will be beneficial for investigating inflammatory disease phenotypes

### Example 4: Effects of LL-37 and its synthetic derivatives on suppression of IL-32-induced pro-inflammatory protein production in human peripheral blood mononuclear cells.

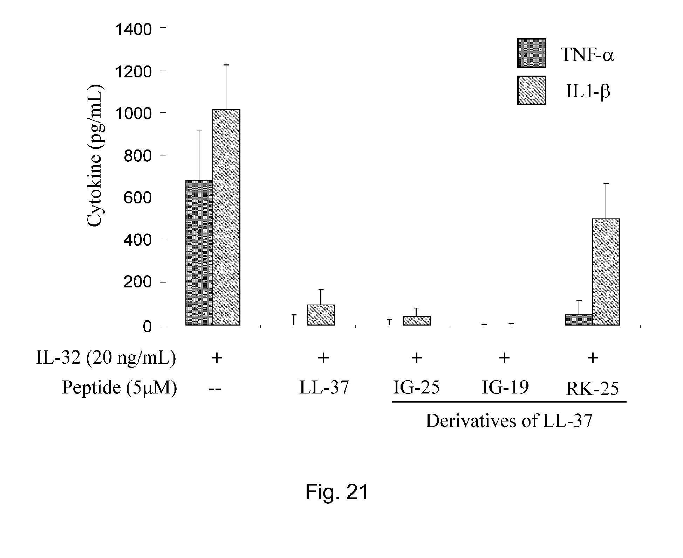

The studies disclosed herein demonstrate that the innate immune-modulatory cationic peptide, human cathelicidin LL-37, and its derivatives can suppress chronic inflammatory cytokine IL-32-induced TNF-α and IL-1β production in macrophage-like THP-1 cell line. In order to confirm these activities in a physiologically relevant system, we evaluated the impact of the peptide in an *ex-vivo* assay using human PBMC, because cells recruited from the circulation are a major component of inflammatory infiltrates. Venous blood was collected from healthy volunteers with informed consent in accordance to a protocol approved by the Institutional Review Board at the University of Manitoba. PBMC was isolated from blood following the teaching of Mookherjee et al. (2009, Systems biology evaluation of immune responses induced by human host defence peptide LL-37 in mononuclear cells. Mol. Biosyst. 5:483-496). PBMC were stimulated with chronic inflammatory cytokine IL-32 (20 ng/ml) in the presence and absence of 5 µM of peptides, LL-37 and its derivatives (Table 7) for 24 hr. The tissue culture supernatants were monitored for the production of pro-inflammatory cytokines TNF-α and IL-1β by ELISA. Fig. 21 shows that the host defence peptide LL-37 and derivatives IG-25 and IG-19 neutralized the production of IL-32-induced both TNF-α and IL-1β (*p* ≤ 0.05). In contrast, RK-25 significantly suppressed IL-32-induced TNF-α production by > 90%, but not IL-1β production (Fig. 22). Results shown are an average of four independent biological experiments performed with cells isolated from blood of four different donors ± standard error. These results suggest that LL-37 and its derivatives IG-25 and IG-19 can significantly neutralize chronic inflammatory cytokine IL-32-induced pro-inflammatory proteins TNF-α and IL-1β production in human blood-derived mononuclear cells.

**Table 7:**

| **Peptide** | **SEQ ID NO:** | **Sequence** | **Mol wt** | **5µM** |
|---|---|---|---|---|
| LL-37 | SEQ ID NO:21 | LLGDFFRKSKEKIGKEFKRIVQRIKDFLRNLVPRTES | 4493.33 | 22.46 µg/ml |
| IG-25 | SEQ ID NO:22 | IGKEFKRIVQRIKDFLRNLVPRTES-NH₂ | 3043 | 15.22 µg/ml |
| IG-19 | SEQ ID NO:23 | IGKEFKRIVQRIKDFLRNL-NH₂ | 2374 | 11.87 µg/ml |
| RK-25 | SEQ ID NO:24 | RKSKEKIGKEFKRIVQRIKDFLRNL-NH₂ | 3131 | 15.6 µg/ml |

## Claims

1. A composition for treatment of a chronic inflammation condition or an autoimmune disorder induced by cytokine IL-32, the composition comprising a therapeutically effective amount of a polypeptide molecule consisting an amino acid sequence selected from the group consisting of SEQ ID NO:22 and SEQ ID NO:23, and a pharmaceutically acceptable carrier.

2. A composition according to claim 1, wherein the polypeptide molecule consists of SEQ ID NO:22.

3. A composition according to claim 1, wherein the polypeptide molecule consists of SEQ ID NO:23.

4. The composition of any of claims 1-3, for use in the treatment of a chronic inflammation condition or an autoimmune disorder induced cytokine IL-32.

5. the composition according to claim 4, wherein the chronic inflammation condition is an arthritis condition.

6. An isolated protein for treatment of a chronic inflammation condition or an autoimmune disorder induced cytokine IL-32, the isolated protein comprising a polypeptide molecule consisting of an amino acid sequence selected from the group consisting of SEQ ID NO: 22 and SEQ ID NO 23.

7. An isolated protein according to claim 6, wherein the polypeptide molecule consists of SEQ ID NO: 22.

8. An isolated protein according to claim 6, wherein the polypeptide molecule consists of SEQ ID NO:23.

9. An isolated nucleic acid encoding the protein of claim 7,

10. An isolated nucleic acid according to claim 9, operable linked to a promoter.

11. An isolated nucleic acid encoding the protein of claim 8.

12. An isolated nucleic acid of claim 11, operably linked to a promoter.


SEQUENCE LISTING

<110>  University of Manitoba

<120>  Modulation of cytokine-induced chronic inflammatory responses

<130>  P6042809EP1

<140>  EP11746785.2
<141>  2012-09-21

<140>  PCT/CA2011/000207
<141>  2011-02-25

<150>  61/308,269
<151>  2010-02-25

<150>  61/362,935
<151>  2010-07-09

<160>  24

<170>  PatentIn version 3.5

<210>  1
<211>  21
<212>  DNA
<213>  Artificial Sequence

<220>
<223>  primer

<400>  1
cagcctcttc tccttcctga t                                                21


<210>  2
<211>  20
<212>  DNA
<213>  Artificial Sequence

<220>
<223>  primer

<400>  2
gccagagggc tgattagaga                                                  20


<210>  3
<211>  20
<212>  DNA
<213>  Artificial Sequence

<220>
<223>  primer

<400>  3
tcctgcatcc cccatagtta                                                  20


<210>  4
<211>  20
<212>  DNA

<213>  Artificial Sequence

<220>
<223>  primer

<400>  4
cttcaggaac agccaccagt                                                   20


<210>  5
<211>  20
<212>  DNA
<213>  Artificial Sequence

<220>
<223>  primer

<400>  5
caggagccca gctatgaact                                                   20


<210>  6
<211>  20
<212>  DNA
<213>  Artificial Sequence

<220>
<223>  primer

<400>  6
caggagccca gctatgaact                                                   20


<210>  7
<211>  20
<212>  DNA
<213>  Artificial Sequence

<220>
<223>  primer

<400>  7
agacagcaga gcacacaagc                                                   20


<210>  8
<211>  19
<212>  DNA
<213>  Artificial Sequence

<220>
<223>  primer

<400>  8
aggaaggctg ccaagagag                                                    19


<210>  9
<211>  20
<212>  DNA
<213>  Artificial Sequence

<220>

<223>  primer

<400>  9
agcttcatgc ctccctactg                                                    20


<210>  10
<211>  20
<212>  DNA
<213>  Artificial Sequence

<220>
<223>  primer

<400>  10
ctgctgagtc tcccagtggt                                                    20


<210>  11
<211>  22
<212>  DNA
<213>  Artificial Sequence

<220>
<223>  primer

<400>  11
gatctgtgtc cttcaccatg ag                                                 22


<210>  12
<211>  20
<212>  DNA
<213>  Artificial Sequence

<220>
<223>  primer

<400>  12
aaacagccat cacagacgaa                                                    20


<210>  13
<211>  21
<212>  DNA
<213>  Artificial Sequence

<220>
<223>  primer

<400>  13
cccagttgaa gaacccatag c                                                  21


<210>  14
<211>  22
<212>  DNA
<213>  Artificial Sequence

<220>
<223>  primer

<400>  14

cgaggaacat tcatgatgtc ag                                                22

<210>  15
<211>  20
<212>  DNA
<213>  Artificial Sequence

<220>
<223>  primer

<400>  15
gtaacccgtt gaaccccatt                                                    20

<210>  16
<211>  20
<212>  DNA
<213>  Artificial Sequence

<220>
<223>  primer

<400>  16
ccatccaatc ggtagtagcg                                                    20

<210>  17
<211>  20
<212>  DNA
<213>  Artificial Sequence

<220>
<223>  primer

<400>  17
ctggcagctc ttctcaaagc                                                    20

<210>  18
<211>  21
<212>  DNA
<213>  Artificial Sequence

<220>
<223>  primer

<400>  18
tccaggtcat agagaggctc a                                                  21

<210>  19
<211>  19
<212>  DNA
<213>  Artificial Sequence

<220>
<223>  primer

<400>  19
cagaacaacc tgcagcaga                                                     19

<210>  20
<211>  20
<212>  DNA
<213>  Artificial Sequence

<220>
<223>  primer

<400>  20
tcagcaattt ctggctggtt                                                    20


<210>  21
<211>  37
<212>  PRT
<213>  Homo sapiens

<400>  21

Leu Leu Gly Asp Phe Phe Arg Lys Ser Lys Glu Lys Ile Gly Lys Glu
1               5                   10                  15


Phe Lys Arg Ile Val Gln Arg Ile Lys Asp Phe Leu Arg Asn Leu Val
            20                  25                  30


Pro Arg Thr Glu Ser
        35


<210>  22
<211>  25
<212>  PRT
<213>  Artificial Sequence

<220>
<223>  LL-27 synthetic derivative

<400>  22

Ile Gly Lys Glu Phe Lys Arg Ile Val Gln Arg Ile Lys Asp Phe Leu
1               5                   10                  15


Arg Asn Leu Val Pro Arg Thr Glu Ser
            20                  25


<210>  23
<211>  19
<212>  PRT
<213>  Artificial Sequence

<220>
<223>  LL-37 synthetic derivative

<400>  23

Ile Gly Lys Glu Phe Lys Arg Ile Val Gln Arg Ile Lys Asp Phe Leu
1               5                   10                  15

Arg Asn Leu

<210> 24
<211> 25
<212> PRT
<213> Artificial Sequence

<220>
<223> LL-37 synthetic derivative

<400> 24

Ile Gly Lys Glu Phe Lys Arg Ile Val Gln Arg Ile Lys Asp Phe Leu
1               5                   10                  15

Arg Asn Leu Val Pro Arg Thr Glu Ser
            20                  25